# EUROPEAN PATENT APPLICATION

(11) **EP 1 988 089 A1**
(43) Date of publication of application: **05.11.2008**
(21) Application number: 08013422.4
(22) Date of filing: 26.10.2007
(51) Int. Cl.: C07D 401/04, A61K 31/506, A61P 35/00

(54) **Imatinib base, and imatinib mesylate and processes for preparation thereof**

(30) Priority: 26.10.2006 US 854774 P; 22.11.2006 US 860624 P; 11.12.2006 US 874420 P; 14.06.2007 US 934911 P; 05.07.2007 US 958367 P; 02.08.2007 US 963238 P; 05.09.2007 US 967617 P; 25.09.2007 US 995332 P; 05.10.2007 US 997849 P; 11.10.2007 US 979256 P
(62) Divisional of application: 07839811.2
(71) Applicant: Sicor, Inc., Irvine, CA 92618 (US)
(72) Inventor: Macdonald, Peter Lindsay, Gentilino (CH); Rossetto, Pierluigi, Balerna (CH); Jegorov, Alexandr, 373 16 Dobra Voda (CH); Giolito, Andrea, 20152 Milan (IT); Tentorio, Dario, Vigano (LC) (IT); Canavesi, Augusto, 22070 Locate Varesino (IT); Gavenda, Ales, 725 28 Ostrava - Lhotka (CZ)
(74) Representative: Wong, Kit Yee

(57) **Abstract**

The present invention provides amorphous imatinib base and a process for its preparation.

## Description

### CROSS REFERENCE TO RELATED APPLICATIONS

The present application claims the benefit of the following United States Provisional Patent Application Nos.: 60/854,774, filed October 26, 2006; 60/874,420, filed December 11, 2006; 60/958,367, filed July 5, 2007; 60/963,238, filed August 2, 2007; 60/967,617, filed September 5, 2007; 60/995,332, filed September 25, 2007; 60/860,624, filed November 22, 2006; 60/979,256, filed October 11, 2007; 60/934,911, filed June 14, 2007; and 60/TBA (Attorney Docket No. 13760/A403P2), filed October 5, 2007. The contents of these applications are incorporated herein by reference.

### FIELD OF INVENTION

The present invention is directed to crystalline Imatinib base, Imatinib free of desmethyl imatinib and imatinib mesylate free of desmethyl Imatinib mesylate, respectively, processes for preparation thereof and pharmaceutical compositions thereof.

### BACKGROUND OF THE INVENTION

Imatinib mesylate, 4-(4-methylpiperazin-1-ylmethyl)-N-[4-methyl-3-[(4-pyrinin-3-yl)pyrimidin-2-yloamino]phenyl]benzamide mesylate, a compound having the following chemical structure, 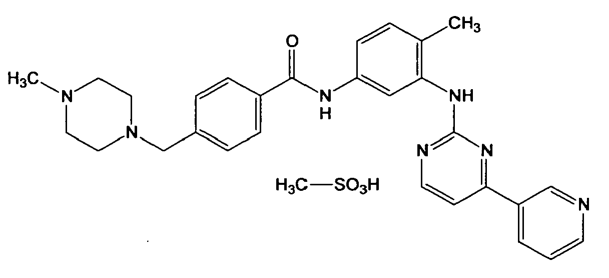 is a protein-tyrosine kinase inhibitor, especially useful in the treatment of various types of cancer and can also be used for the treatment of atherosclerosis, thrombosis, restenosis, or fibrosis. Thus, imatinib mesylate can be used also for the treatment of non-maligant diseases. Imatinib mesylate is usually administered orally in the form of a suitable salt, e.g., in the form of imatinib mesylate, and is marketed by Novartis under the trade name Gleevec^{®} in the USA.

Imatinib base is the key intermediate for preparing imatinib salts such as imtainib mesylate. US patent No. 5521184, International Application Nos. WO 03/066613, 04/108699, 04/074502, 06/071130 and US application Nos. 04/0248918, 06/0149061, 06/0223817 describe the synthesis of Imatinib-base, its isolation and its purification by column chromatography or by crystallization from different solvents.

The isolation is done by precipitating the base from mixtures of n-butanol and butylacetate, ethylacetate, water, or mixtures of water and organic solvent. The isolated crystalline form of imatinib base described in the above references is characterized by main PXRD peaks at: 6.0, 17.2, 18.1, 18.7, 19.8, 20.9, 23.8, 24.3, and 25.2 ± 0.2 degrees two-theta, denominated form I. Specifically such isolation as described includes "mixing Imatinib base and n-butanol, heating the mixture to 91 °C until a clear solution is obtained. The solution is cooled to room temperature and the resulting crystals are washed with 2 ml of cold n-butanol, filtered and dried under reduced pressure." The same process is also reported for the following solvents: toluene, cyclohexane, chloroform, dichloromethane, acetonitrile, methanol, methylethyl-ketone, methyl- iso-butyl- ketone, iso-propanol and ethylacetate; wherein the temperature for achieving a clear solution is different. Further, the column chromatography is done by using methanol or its mixtures with chloroform. When purified by crystallization, the solvent of choice can be n-butanol, toluene and others.

The present invention relates to the solid state physical properties of Imatinib base. These properties can be influenced by controlling the conditions under which imatinib base is obtained in solid form. Solid state physical properties include, for example, the flow-ability of the milled solid. Flow-ability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular polymorphic form of a substance. The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetry (DSC) and can be used to distinguish some polymorphic forms from others. A particular polymorphic form may also give rise to distinct spectroscopic properties that may be detectable by powder X-ray crystallography (PXRD), solid state ¹³C NMR spectrometry and infrared spectroscopy.

One of the most important physical properties of a pharmaceutical compound, which can form polymorphs, is its solubility in aqueous solution, particularly the solubility in gastric juices of a patient. Other important properties relate to the ease of processing the form into pharmaceutical dosages, as the tendency of a powdered or granulated form to flow and the surface properties that determine whether crystals of the form will adhere to each other when compacted into a tablet.

Further, the base can then be converted to the mesylate salt, which is isolated by precipitation from the reaction mixture consisting of the base, methansulfonic acid and a solvent as described in International Application Nos. WO 99/03854, WO 2005/077933, WO 2005/095379, WO 2004/106326, WO 2006/054314, WO 2006/024863, WO 2006/048890, US2006/0030568, WO 2007/023182, and US Patent No. 6,894,051.

Like any synthetic compound, Imatinib mesylate can contain extraneous compounds or impurities, such as desmethyl imatinib mesylate. Des-methyl imatinib mesylate and process for its preparation are disclosed in US Patent No. 7081532.

Impurities in Imatinib mesylate, or any active pharmaceutical ingredient ("API"), are undesirable and, in extreme cases, might even be harmful to a patient being treated with a dosage form containing the API.

The purity of an API produced in a manufacturing process is critical for commercialization. The U.S. Food and Drug Administration ("FDA") requires that process impurities be maintained below set limits. For example, in its ICH Q7A guidance for API manufacturers, the FDA specifies the quality of raw materials that may be used, as well as acceptable process conditions, such as temperature, pressure, time, and stoichiometric ratios, including purification steps, such as crystallization, distillation, and liquid-liquid extraction. *See* ICH Good Manufacturing Practice Guide for Active Pharmaceutical Ingredients, Q7A, Current Step 4 Version (November 10, 2000).

The product of a chemical reaction is rarely a single compound with sufficient purity to comply with pharmaceutical standards. Side products and by-products of the reaction and adjunct reagents used in the reaction will, in most cases, also be present in the product. At certain stages during processing of an API, it must be analyzed for purity, typically, by high performance liquid chromatography ("HPLC") or thin-layer chromatography ("TLC"), to determine if it is suitable for continued processing and, ultimately, for use in a pharmaceutical product. The FDA requires that an API is as free of impurities as possible, so that it is as safe as possible for clinical use. For example, the FDA recommends that the amounts of some impurities be limited to less than 0.1 percent. *See* ICH Good Manufacturing Practice Guide for Active Pharmaceutical Ingredients, Q7A, Current Step 4 Version (November 10, 2000).

Generally, side products, by-products, and adjunct reagents (collectively "impurities") are identified spectroscopically and/or with another physical method, and then associated with a peak position, such as that in a chromatogram, or a spot on a TLC plate. *See* Strobel, H.A., et al., CHEMICAL INSTRUMENTATION: A SYSTEMATIC APPROACH, 953, 3d ed. (Wiley & Sons, New York 1989). Once a particular impurity has been associated with a peak position, the impurity can be identified in a sample by its relative position in the chromatogram, where the position in the chromatogram is measured in minutes between injection of the sample on the column and elution of the impurity through the detector. The relative position in the chromatogram is known as the "retention time."

As is known by those skilled in the art, the management of process impurities is greatly enhanced by understanding their chemical structures and synthetic pathways, and by identifying the parameters that influence the amount of impurities in the final product.

The discovery of new polymorphic forms of Imatinib base provides a new opportunity to improve the performance of the synthesis of the active pharmaceutical ingredient (API), Imatinib mesylate, by producing crystalline forms of Imatinib base having improved characteristics, such as flowability, and solubility. Thus, there is a need in the art for polymorphic forms of Imatinib base. In addition, providing Imatinib free of des-methyl Imatinib, and Imatinib mesylate free of des-methyl Imatinib mesylate, and means for preparation thereof is beneficial.

### SUMMARY OF THE INVENTION

In one embodiment, the present invention encompasses crystalline Imatinib base characterized by data selected from the group consisting of: a powder XRD pattern having any five peaks selected from the list consisting of peaks at about: 6.4, 8.1, 10.2, 12.8, 16.1, 19.4, 20.4, 21.7, 22.1, 25.8 and 26.7 ± 0.2 degrees two-theta; a solid-state ¹³C NMR spectrum with signals at about 159.6, 146.7, 136.8 and 132.4 ± 0.2 ppm; a solid-state ¹³C NMR spectrum having chemical shift differences between the signal exhibiting the lowest chemical shift and another in the chemical shift range of 100 to 180ppm of about 51.2, 38.3, 28.4 and 24.0 ± 0.1 ppm, and combinations thereof.

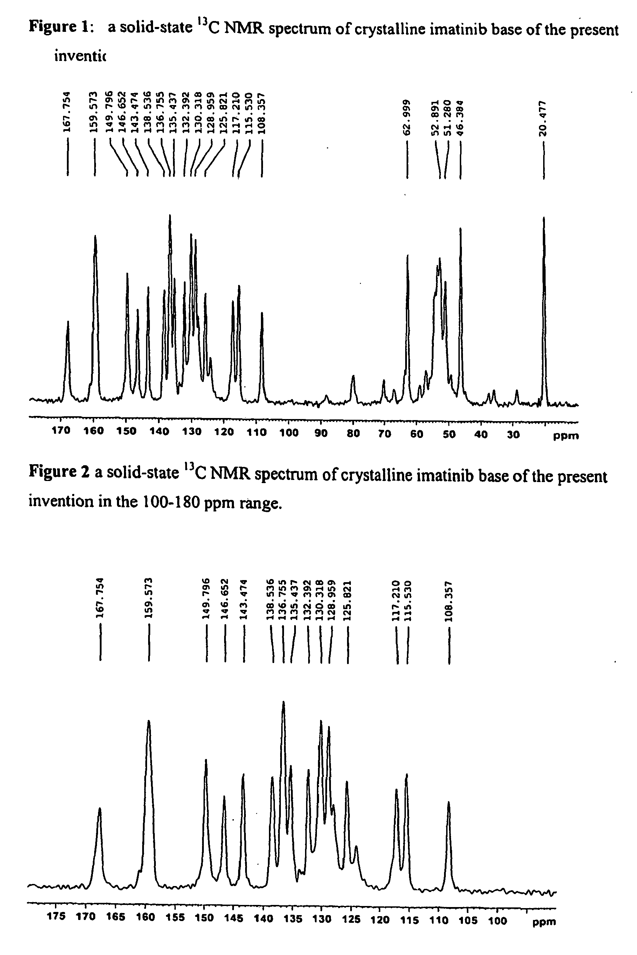

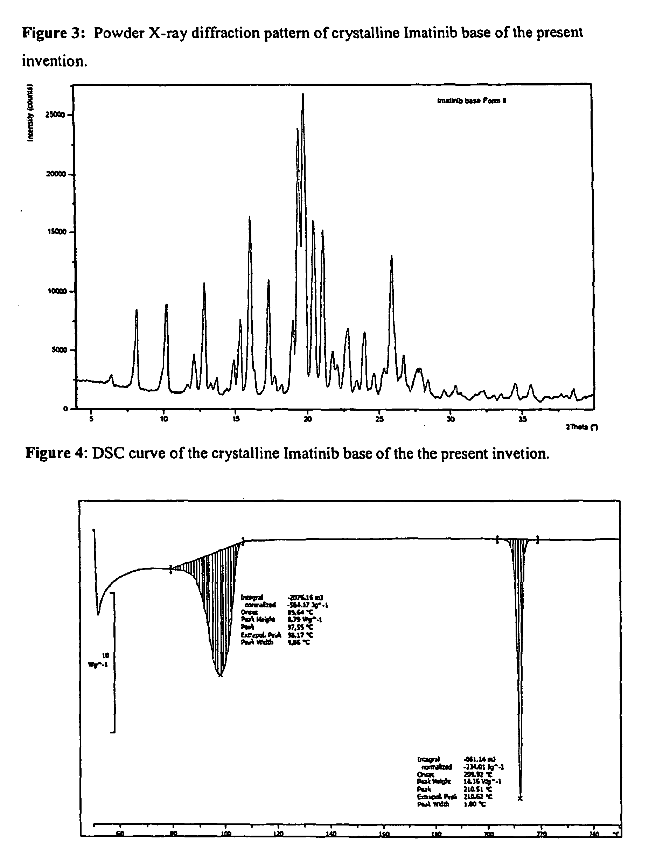

In another embodiment, the present invention encompasses crystalline Imatinib base characterized by data selected from the group consisting of: a powder XRD depicted in figure 3, a solid state ¹³C NMR spectrum depicted in figure 1, and a solid state ¹³C NMR spectrum depicted in figure 2.

In yet another embodiment, the present invention encompasses a process for preparing the above crystalline Imatinib base comprising crystallizing imatinib base from a mixture containing pyridine.

In one embodiment, the present invention encompasses amorphous imatinib base. Amorphous imatinib base has a large surface area and a faster dissolution rate than the crystalline imatinib base. These properties of amorphous imatinib base are advantageous in a subsequent reaction with methanesulfonic acid to prepare imatinib mesytlate.

In another embodiment, the present invention encompasses a process for preparing amorphous Imatinib base comprising lyophilizing a solution of imatinib base in 1,4-dioxane.

In yet another embodiment, the present invention encompasses a process for preparing Imatinib salt comprising preparing any one of the forms of imatinib base of the present invention, and converting them to Imatinib salt.

In one embodiment, the present invention encompasses the use of any one of the forms of imatinib base of the present invention for the preparation of Imatinib salt.

In another embodiment, the present invention encompasses Imatinib of the following formula 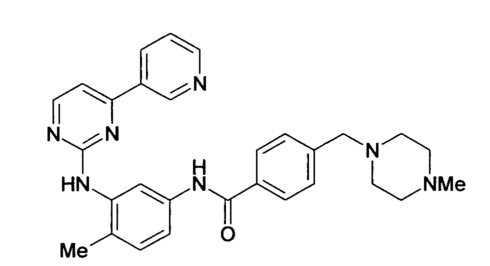 having less than about 0.09% by HPLC area percent units of desmethyl-imatinib of the following formula.

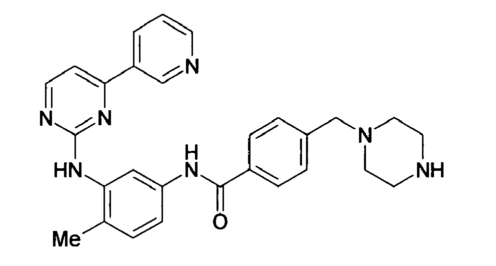

In one embodiment, the present invention encompasses a process for preparing Imatinib base having less than about 0.09% by HPLC area percent units of desmethyl-imatinib comprising measuring the level of the desmethyl impurity of formula II 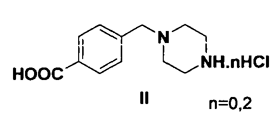 in at least one batch of the compound of formula I 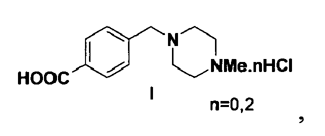 selecting a batch of the compound of formula I having less than about 0.15% by HPLC area percent units of the desmethyl impurity of formula II; and preparing imatinib base comprising the selected batch of the compound of formula I.

In one embodiment, the present invention encompasses a process for preparing Imatinib having less than about 0.09% by HPLC area percent units of desmethyl-imatinib comprising:
a) providing the compound of formula I 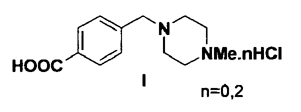 having less than about 0.15% by HPLC area percent units of the desmethyl compound of formula II; 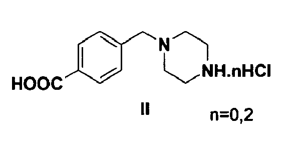
b) reacting it with the amine of formula III, 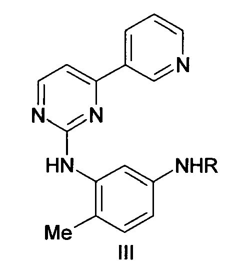 to obtain Imatinib having less than about 0.09% by HPLC area percent units of desmethyl imatinib, and optionally
c) crystallizing imatinib base to obtain crystalline Imatinib having less than about 0.09% by HPLC area percent units of desmethyl imatinib.

In another embodiment, the present invention encompasses a process for preparing Imatinib mesylate having less than about 0.09% by HPLC area percent units of desmethyl-imatinib mesylate comprising measuring the level of the desmethyl-imatinib in at least one batch of imatinib, selecting a batch of imatinib having less than about 0.09% by HPLC area percent units of desmethyl-imatinib; and preparing imatinib mesylate comprising the selected batch of imatinib base.

In yet another embodiment, the present invention encompasses Imatinib mesylate of the following formula 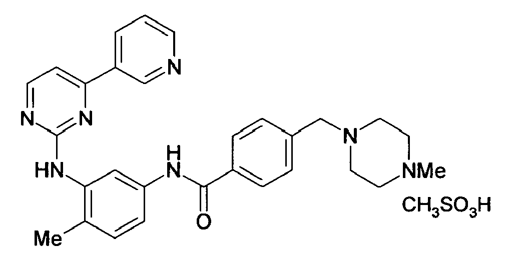 having less than about 0.09% by HPLC area percent units of desmethyl-imatinib mesylate of the following formula.

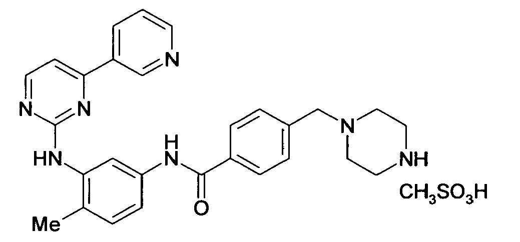

In another embodiment, the present invention encompasses a pharmaceutical composition comprising imatinib mesylate having less than about 0.09% by HPLC area percent units of desmethyl-imatinib mesylate and at least one pharmaceutically acceptable excipient.

In yet another embodiment, the present invention encompasses a process for preparing the pharmaceutical composition, comprising combining imatinib mesylate having less than about 0.09% by HPLC area percent units of desmethyl-imatinib mesylate and the pharmaceutically acceptable excipient.

In one embodiment, the present invention encompasses the use of imatinib mesylate having less than about 0.09% by HPLC area percent units of desmethyl-imatinib mesylate in the manufacture of a pharmaceutical composition for the treatment of various types of cancer, atherosclerosis, thrombosis, restenosis, or fibrosis.

In another embodiment, the present invention encompasses the des-methyl compound of formula IV

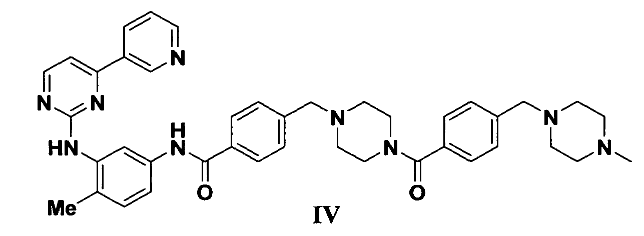

In yet another embodiment, the present invention encompasses a process for preparing the des-methyl compound of formula IV comprising reacting des-methyl imatinib of the following formula 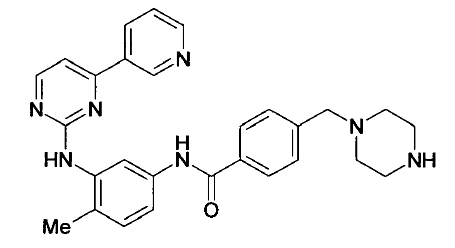 and the compound of formula V, 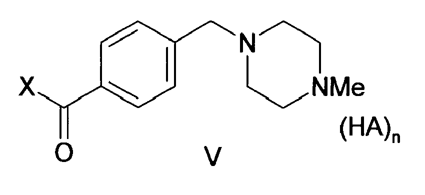 wherein X is a leaving group, HA is an acid, and n is 0, 1 or 2.

In one embodiment, the present invention encompasses a process of determining the presence of the des-methyl compound of formula IV in Imatinib by a process comprising carrying out HPLC or TLC with the des-methyl compound of formula IV as a reference marker.

In another embodiment, the present invention encompasses a process of determining the amount of the des-methyl compound of formula IV in a sample comprising the des-methyl compound of formula IV and imatinib by a process comprising carrying out HPLC with the des-methyl compound of formula IV as a reference standard.

### BRIEF DESCRIPTION OF THE DRAWINGS

Figure 1 illustrates a solid-state ¹³C NMR spectrum of crystalline Imatinib base of the present invention.
Figure 2 illustrates a solid-state ¹³C NMR spectrum of the above crystalline Imatinib base in the range of 100-180 ppm.
Figure 3 illustrates a powder X-ray diffraction pattern of the above crystalline Imatinib base.
Figure 4 illustrates DSC curve of the above crystalline Imatinib base.
Figure 5 illustrates a powder X-ray diffraction pattern of amorphous Imatinib base.
Figure 6 illustrates ¹HNMR (DMSO-d₆) spectrum of des-methyl impurity of formula IV.
Figure 7 illustrates ¹³CNMR (DMSO-d₆) spectrum of des-methyl impurity of formula IV.
Figure 8 illustrates IR spectrum of des-methyl impurity of formula IV.
Figure 9 illustrates MS spectrum of des-methyl impurity of formula IV.

### DETAILED DESCRIPTION OF THE INVENTION

As used herein, the term "Imatinib" refers to Imatinib base of the following formula.

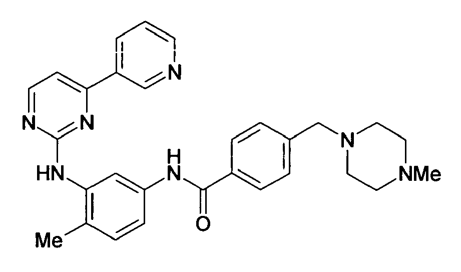

As used herein, the term "chemical shift difference" refers to the difference in chemical shifts between a reference signal and another signal in the same solid-state ¹³C NMR spectrum. In the present patent application the chemical shift differences were calculated by subtracting the chemical shift value of the signal exhibiting the lowest chemical shift (reference signal) in the solid-state ¹³C NMR spectrum in the range of 90 to 180 ppm from chemical shift values of another (observed) signals in the same solid-state NMR spectrum in the range of 90 to 180 ppm. These chemical shift differences are to provide a measurement for a substance, for example imatinib, of the present invention compensating for a phenomenon in NMR spectroscopy wherein, depending on the instrumentation, temperature, and calibration method used, a shift in the solid-state NMR "fingerprint" is observed. This shift in the solid-state NMR "fingerprint", having signals at certain positions, is such that although the individual chemical shifts of signals have altered, the difference between chemical shifts of each signal and another is retained.

The present invention encompasses crystalline Imatinib base characterized by data selected from the group consisting of at least one of : a powder XRD pattern having any five peaks selected from the list consisting of peaks at about: 6.4, 8.1, 10.2, 12.8, 16.1, 19.4, 20.4, 21.7, 22.1, 25.8 and 26.7 ± 0.2 degrees two-theta; a solid-state ¹³C NMR spectrum with signals at about 159.6, 146.7, 136.8 and 132.4 ± 0.2 ppm; a solid-state ¹³C NMR spectrum having chemical shift differences between the signal exhibiting the lowest chemical shift and another in the chemical shift range of 100 to 180ppm of about 51.2, 38.3, 28.4 and 24.0 ± 0.1 ppm. The signal exhibiting the lowest chemical shift in the chemical shift range of 100 to 180 ppm is, typically, at about 108.4± 0.2 ppm.

This crystalline form can also be characterized by data selected from the group consisting of: a powder XRD depicted in figure 3, a solid state ¹³C NMR spectrum depicted in figure 1, and a solid state ¹³C NMR spectrum depicted in figure 2.

This crystalline Imatinib base can be further characterized by data selected from the group consisting of: a powder XRD pattern having peaks at about 8.1, 10.2, 12.8, 16.1, and 19.4 ± 0.2 degrees two-theta; a powder XRD pattern having peaks at about 6.4, 8.1, 10.2, 19.4, 20.4 and 25.8 ± 0.2 degrees two-theta; a powder XRD pattern with peaks at about 17.3, 20.4, 21.1, and 25.8 ± 0.2 degrees two-theta; a powder XRD pattern with peaks at about 6.4, 21.7, 22.1 and 26.7± 0.2 degrees two-theta; a solid-state ¹³C NMR spectrum with signals at about 125.8 and 108.4 ± 0.2 ppm; a DSC curve having 2 peaks, the first peak is an endothermic peak at 97.6°C due to desolvatation of the solvate, the second is an endothermic peak at 210.5 °C due to melting of desolvated product; and a DSC curve depicted in figure 4.

The crystalline imatinib base of the present invention is a pyridine solvate of imatinib base, preferably, a hemi-pyridine solvate. The content of pyridine is of about 7% (w/w) as measured by Gas Chromatography (GC).

The said crystalline of imatinib base has less than about 20% of crystalline form I of Imatinib base, preferably, less than about 10% of crystalline form I, most preferably, less than about 5% of crystalline form I, as measured either by PXRD or solid-state ¹³C NMR. Typically, the content of crystalline form I of imatinib base in the above form is measured by % by weight.

The content of crystalline form I can be measured, for example, by PXRD or by solid state ¹³C NMR. When measured by PXRD, the content of crystalline form I can be determined by using peaks of crystalline form I that are selected from the following list of peaks: 6.0, 9.5, 14.0, 17.1, 18.1, 18.6, 24.2 and 29.1 ± 0.2 degrees two theta.

When measured by solid-state ¹³C NMR, the content of crystalline form I can determined by using signals selected from the following list of signals at about: 105.4, 122.2, 124.2, 129.1, 140.0, 142.4, 148.5, 150.7, 158.4 and 162.2 ppm ± 0.2 ppm.

The said crystalline Imatinib base may be prepared according to the process disclosed in Co-application no. 11/...,... filed October 26, 2007 (Attorney Docket No. 13150/A400US1), incorporated herein by reference. The process comprises reacting the amine of formula III, 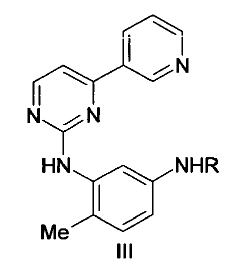 with a 4-[(4-methyl-1-piperazinyl)methyl]benzoyl derivative of formula V 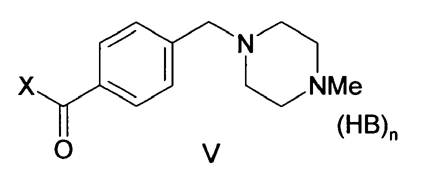 and an amount of about 2 to about 10, preferably about 4 to about 7, and most preferably about 5 to about 6 volumes of pyridine per gram of the compound of formula III, and recovering Imatinib base; wherein X is a leaving group selected from the group consisting of: Cl, Br, preferably, Cl; wherein R is either H or an alkyl group, preferably, H; n= 0, 1, or 2, preferably n=0 or 2; and HB is an acid, preferably HB is HCl, HI or HBr, more preferably, HB is HCl. Recovering imatinib base provides the crystalline imatinib base of the present invention. The alkyl group is preferably a C₁-C₆ alkyl group.

The crystalline Imatinib base of the present invention can also be prepared by a process comprising crystallizing imatinib base from a mixture containing pyridine.

The mixture containing pyridine may include a solvent, an anti-solvent and pyridine. The crystallization is performed by a process comprising providing a solution containing imatinib base or salt thereof, pyridine and the said solvent, and adding an anti-solvent to obtain a precipitate of the said crystalline imatinib base; wherein when imatinib salt is used, the mixture comprises also an additional base.

Typically, said solvent includes a solvent that dissolves pyridine, preferably, water, a water-miscible organic solvent or mixtures thereof. Preferably, the water-miscible organic solvent is selected from the group consisting of: dimethylformamide, dimethylacetamide, tetrahydrofuran, alcohol, acetone, acetonitrile, dioxane, dimethylsulfoxide, and mixtures thereof. Preferably, the alcohol is C₁₋₃ alcohol, more preferably, methanol, ethanol, propanol or isopropanol. More preferably, the solvent is dimethylformamide, dimethylacetamide, tetrahydrofuran, or water, most preferably, water.

The additional base can be an organic base or an inorganic base. Preferably, the organic base is a tertiary amine, such as diisoproylethylamine or triethylamine. A tertiary amine is typically of the formula (N)R₃, where each R is independently selected from a C₁-C₆ alkyl group. Preferably, the inorganic base is sodium or potassium hydroxide, sodium or potassium carbonate, sodium or potassium bicarbonate or ammonia. More preferably, the base is ammonia.

The solution is provided by heating the combination of imatinib base or salt, the said solvent, pyridine and optionally the additional base. Preferably, the combination is heated to a temperature of about 5°C to about 70°C, more preferably, to about 40°C to about 50°C. The Imatinib salt includes but is not limited to Imatinib hydrochloride or Imatinib mesylate. Optionally, when Imatinib salt is used, the process can be done without heating.

Usually, pyridine is present in the solution in an amount of about 2 to about 10 volumes per gram of Imatinib base or salt. The solution can be also characterized by an amount of pyridine of about 50% to about 70% volume per volume of solution

In the process of the present invention, the addition of the anti-solvent to the solution may provide said mixture. Preferably, the mixture is a slurry. The anti-solvent can be the same as the solvent or a different solvent. Preferably, the anti-solvent is water.

Usually, pyridine is present in the slurry in an amount of about 5% to about 30% volumes per the total volume of solvent and anti-solvent combined, more preferably, of about 10% to about 20% per the total volume of solvent and anti-solvent.

The crystallization process may further comprise cooling the slurry, and maintaining the cooled slurry, to increase the yield of the said crystalline Imatinib base. Preferably, cooling may be carried out to a temperature of about 30°C to about 0°C, more preferably, to about 20°C to about 15°C. Preferably, the cooled slurry is maintained for about 1 hour to about 24 hours, more preferably, for about 14 hours to about 16 hours.

The crystallization process may further comprise recovering the precipitated crystalline imatinib base. The recovery may be done by filtration. The recovered product can be dried. Preferably, the drying is done at a temperature below 45°C.

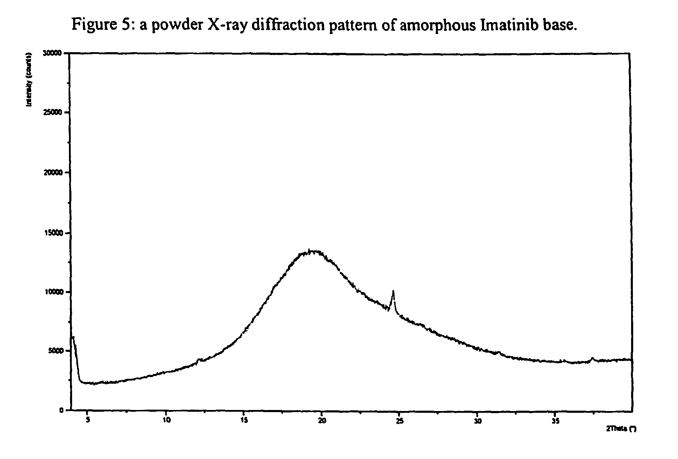

The present invention also encompasses amorphous imatinib base. The amorphous imatinib base can be characterized by the X-ray powder diffraction pattern depicted in Figure 5. Typically, Amorphous imatinib base may be identified by the absence of any significant diffraction peak at the X-ray powder diffraction pattern.

In one embodiment, the amorphous form of the present invention contains less than about 20%, more preferably less than about 10%, and most preferably less than about 5% crystalline form as measured by area percentage XRD.

The amorphous Imatinib base may be prepared by a process comprising lyophilizing a solution of imatinib base in 1,4-dioxane.

Preferably, the solution is provided by combining imatinib base and 1,4-dioxane, and heating the combination. Preferably, heating is conducted to a temperature of about 50°C to about 110°C, more preferably to about 90°C to about 101°C.

Optionally, the solution can be cooled prior to the lyophilization process. Cooling is, preferably, done to a temperature of about 25°C to about 12°C.

The lyophilization process may be done at a temperature of below the freezing temperature of dioxane. Preferably, lyophilization is carried out at a temperature of about 12°C to about 0°C. Lyophilization is preferably done at reduced pressures, preferably, at pressures of about 0.01 to about 100 mBar, more preferably about 0.1 to about 15 mBar, most preferably at about 1mBar.

The present invention encompasses a process for preparing Imatinib salt comprising preparing crystalline or amorphous imatinib base of the present invention, and converting it to imatinib salt. Preferably, the crystalline or amorphous imatinib base is prepared by the processes described herein. Preferably, the Imatinib salt is Imatinib mesylate. The preparation of Imatinib salt from the crystalline or amorphous imatinib base of the present invention may be done by reacting crystalline Imatinib base with an acid. The reaction can be done, for example, according to the process disclosed in International Patent Application. No WO1999/03854.

In such method, the amount of acid that may be used is preferably 1 mole equivalent per mole of the starting imatinib base. This is to avoid the formation of the di-acid salt, which occurs when excess of acid is used, see WO 2005/095379. However, a small excess of acid may be used, such as 0.1 to 0.2 mole equivalent of acid, to ensure completion of transformation of imatinib base to imatinib salt.

When converting the crystalline pyridine solvate discussed above to Imatinib salt, a broader range of the amount of acid, such as 1 mole equivalent to about 1.2 mole equivalent of acid per mole of the starting material crystalline imatinib base, can be used without forming the di-acid salt. The excess of acid, typically, will react with pyridine providing a pyridinium salt, which is soluble in solvents such as alcohols, and remains in the mother liquor while the imatinib salt precipitates.

The above processes preferably prepare a mesylate salt of imatinib. Desmethyl imatinib mesylate of the following formula: 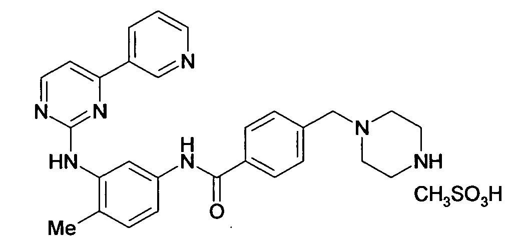 is an impurity of Imatinib mesylate, which is found to be present in the commercial product at levels of at least about 0.09%. The level of the impurity is measured by area percent, preferably, by an HPLC method as described below. Since this impurity is structurally related to Imatinib mesylate, the purification of imatinib mesylate from it is difficult, and purification methods such as crystallization are found to be not efficient for removing it.

In one embodiment, the present invention encompasses Imatinib mesylate of the following formula 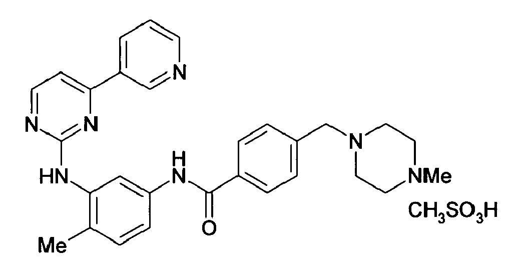 having less than about 0.09% of desmethyl-imatinib mesylate of the following formula.

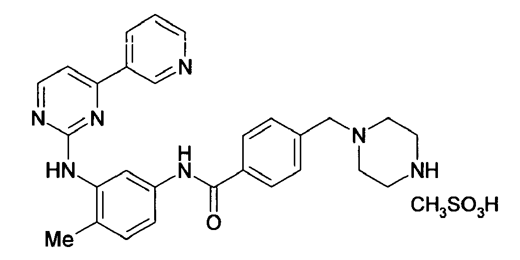

Preferably, Imatinib mesylate has less than 0.07%, more preferably, less than 0.05% of des-methyl imatinib mesylate.

Typically, the measurement of the content of desmethyl-imatinib mesylate is by area percent units, and can be done by an HPLC method comprising:
a) combining a sample comprising of Imatinib mesylate and desmethyl-imatinib mesylate with water to obtain a solution;
b) injecting the solution to a C18 reversed phase silica based HPLC column;
c) eluting the sample from the column using a gradient eluent of a mixture of 1-butanesulfonic acid sodium salt, KH₂PO₄ and H₃PO₄, referred to as mobile phase A, and a mixture of acetonitrile, methanol and tetrahydrofuran, referred to as mobile phase B, and
d) measuring the content of desmethyl Imatinib mesylate using a UV detector.

Desmethyl Imatinib mesylate is a salt of Desmethyl Imatinib. Typically, when transforming Imatinib to imatinib mesylate, by reacting Imatinib with methane sulfonic acid, Desmethyl Imatinib transforms to Desmethyl Imatinib mesylate. In addition, the level of the desmethyl impurity remains similar during this transformation as exemplified in examples 18 and 19. To obtain Imatinib mesylate having less than about 0.09% of desmethyl Imatinib mesylate, the imitanib base starting material for preparing Imatinib mesylate preferably has less than about 0.09% of desmethyl Imatinib.

In another embodiment, the present invention encompasses Imatinib of the following formula 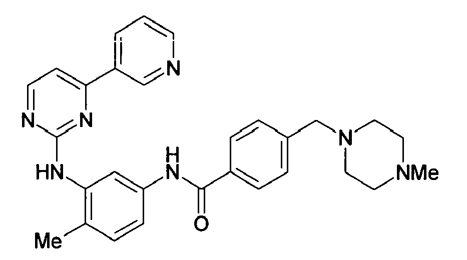 having less than about 0.09% of desmethyl-imatinib of the following formula.

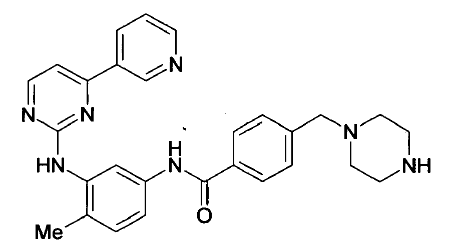

Preferably, Imatinib has less than about 0.07%, more preferably, less than about 0.05% of des-methyl imatinib.

Typically, the measurement of the content of desmethyl-imatinib is by area percent units and can be done by the HPLC method as described above.

The Imatinib having less than about 0.09% of desmethyl-imatinib my be prepared by a process comprising:
a) measuring the level of the desmethyl impurity of formula II 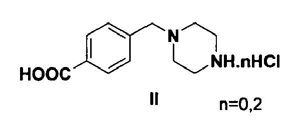 in at least one batch of the compound of formula I, 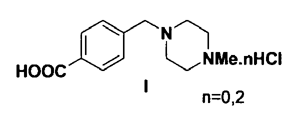
b) selecting a batch of the compound of formula I having less than about 0.15% by HPLC area percent units of the desmethyl impurity of formula II; and
c) preparing imatinib with the selected batch of the compound of formula I, wherein n is either 0, 1 or 2, preferably, 2.

The measurement of the content of the desmethyl impurity of formula II may be by area percent units and can carried out by an HPLC method comprising:
a) combining a sample comprising of the compound of formula I and desmethyl impurity of formula II with water to obtain a solution;
b) injecting the solution to a C18 reversed phase silica based HPLC column;
c) eluting the sample from the column using a gradient eluent of a mixture of 1-butanesulfonic acid sodium salt, KH₂PO₄ and H₃PO₄, referred to as mobile phase A, and a mixture of acetonitrile, referred to as mobile phase B, and
d) measuring the content of the desmethyl impurity of formula II using a UV detector.

The compound of formula I having less than about 0.15% of the desmethyl impurity of formula II is provided by a process comprising crystallizing the compound of formula I from a mixture of water and a C₁₋₃ alcohol.

The crystallization comprises providing a solution of the compound of formula I in a mixture of water and C₁₋₃ alcohol, and precipitating the compound of formula I.

The solution is provided by combining the compound of formula I with a mixture of water and C₁₋₃ alcohol and heating the combination. Preferably, heating is to a temperature of about 55°C to about 80°C, more preferably, about 65 to about 75°C, most preferably about 75°C.

Preferably, the C₁₋₃ alcohol is isopropanol ("IPA"). Preferably, the ratio of the C₁₋₃ alcohol and water in the mixture is of about 80: 20, more preferably, of about 60:50, most preferably, 57:43 v/v, respectively. The starting compound of formula I can be prepared, for example, by the process disclosed in Co-pending U.S. Provisional Application No. 11/...,..., filed October 26, 2007 (Attorney Docket No. 13150/A400US), as described above, such as by reacting a 4-benzoic acid derivative of the following formula, where X is a leaving group: 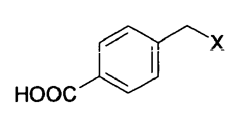 with N-methylpiperazine of the following formula; 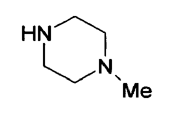 and reacting the product with HCl to obtain the HCl salt.

Typically, the solution is cooled to induce precipitation of the compound of formula I. Preferably, the solution is cooled to a temperature of about 50°C to about - 5°C, more preferably, to about 35°C to about 0°C, most preferably, to about 25°C to about 0°C. The cooling can be done at once or can be done step-wise. When done step-wise, the first cooling is to a temperature of about 35°C to about 15°C, more preferably, to about 25°C to about 20°C, and the second is to a temperature of about 5°C to about -5°C, more preferably, to about 3°C to about 0°C. Preferably, the first cooling stage is done over a period of about 0.5 an hour to about 3 hours, more preferably, for about 1 hour to about 1.5 hours. Preferably, the second cooling is done over a period of about 0.5 an hour to about 5 hours, more preferably, of about 0.5 an hour to about 3 hours, most preferably, of about 1 to about 1.5 hours.

Usually, cooling provides a suspension, which is further maintained at a temperature of about -5°C to about 5°C, preferably at about 3°C to about 0°C, to increase the yield of the compound of formula I. Preferably, the suspension is maintained for about 1 hour to about 5 hours, more preferably, for about 1 hour to about 3 hours, most preferably, for about 1 to about 2 hours. Preferably, the cooling and maintaining steps are done while stirring.

The precipitated compound of formula I is then recovered by any method known to a skilled artisan, such as filtering and drying.

The provided compound of formula I having less than about 0.15% of the desmethyl impurity of formula II is then reacted with the amine of formula III, 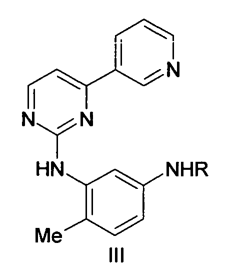 to obtain Imatinib having less than about 0.09% of desmethyl imatinib.

The reaction between the compound of formula I and the amine of formula III can be done, for example, by the process disclosed in U.S. Provisional Application No. 11/...,..., filed October 26, 2007 (Attorney Docket No. 13150/A400US).

The process comprises reacting the amine of formula III, 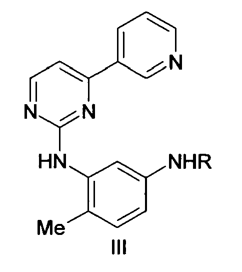 with a 4-[(4-methyl-1-piperazinyl)methyl]benzoyl derivative of formula V 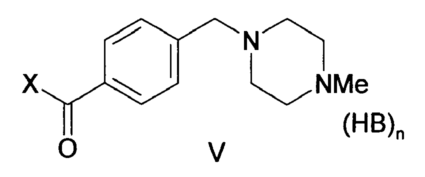 and an amount of about 2 to about 10 volumes (7 to 35 equivalents), more preferably about 4 to about 7 volumes, and most preferably about 5 to about 6 volumes of pyridine per gram of the compound of formula III to obtain Imatinib; and
recovering the obtained Imatinib;
wherein n is 0, 1, or 2; X is a leaving group selected from the group consisting of: Cl, and Br, preferably X is Cl; R is either H or a C₁₋₆ alkyl, preferably, H, and HB is an acid selected from the group consisting of: HCl, HBr, HI, Methanesulfonic acid, and para-toluenesulofinic acid, preferably HB is HCl. The process further includes activating the compound of formula I to obtain the activated acid derivative compound of formula V by reacting the compound of formula I, 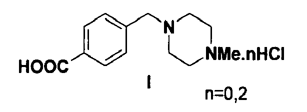 with a carboxylic acid activating agent in the presence of a base or a water absorbing agent to obtain a reaction mixture comprising the compound of formula V. The mixture comprising the compound of formula V is then used in the above process for preparing imatinib.

Alternatively, imatinib having less than about 0.09% of desmethyl-imatinib can be prepared by crystallizing imatinib, or by combining both methods, *i.e.,* providing the compound of formula I having less than about 0.15% of the compound of formula II, and reacting it with the amine of formula III, followed by crystallizing the obtained imatinib. Preferably, the crystallization is carried out by the process as described above.

The obtained imatinib can then be converted to imatinib mesylate having less than about 0.09% of des-methyl imatinib mesylate. The process comprises measuring the level of the desmethyl-imatinib in at least one batch of imatinib, selecting a batch of imatinib having less than about 0.09% of desmethyl-imatinib; and preparing imatinib mesylate with the selected batch of imatinib.

Also, Des-methyl Imatinib can be converted to another impurity, des-methyl compound of formula IV, during the formation of Imatinib base. The des-methyl impurity of formula IV is found to be difficult to purify from imatinib.

In one embodiment, the present invention encompasses the des-methyl compound of formula IV

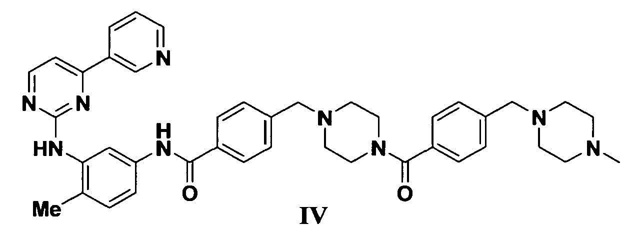

Preferably, the present invention encompasses the isolated des-methyl compound of formula IV (3-{4-[4-(4-Methyl-piperazin-1-ylmethyl)-benzoyl]-piperazin-1-ylmethyl } -N-[4-methyl-3 -(4-pyridin-3-yl-pyrimidin-2-ylamino)-phenyl]-benzamide). As used herein, the term "isolated" when referring to des-methyl compound of formula IV means des-methyl compound of formula IV having not more than about 0.15% of des-methyl imatinib. Typically, the measurement of the content of des-methyl imatinib in the des-methyl compound of formula IV is by area %, preferably, by HPLC.

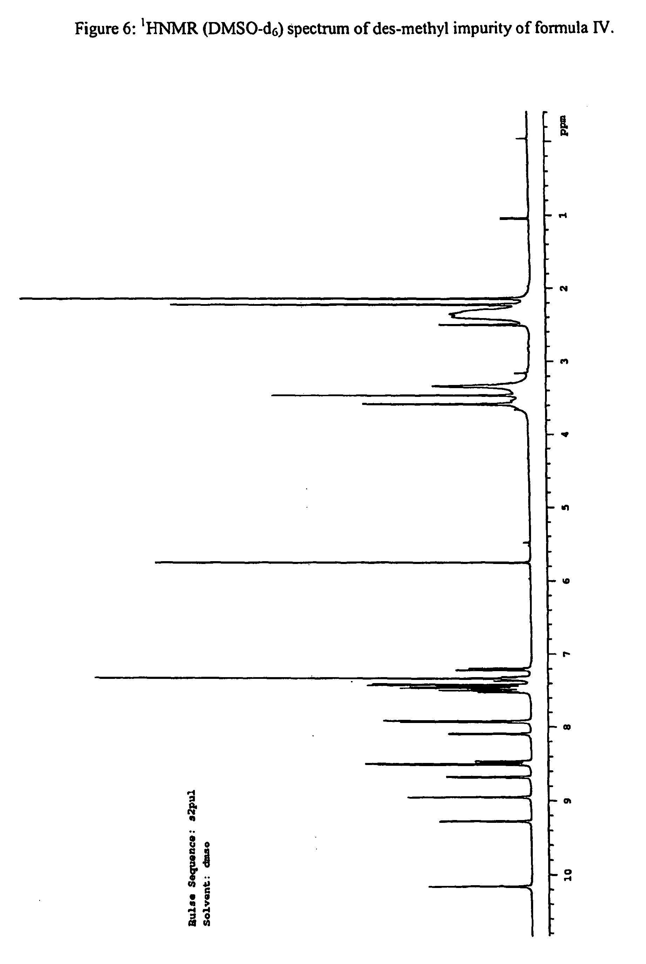

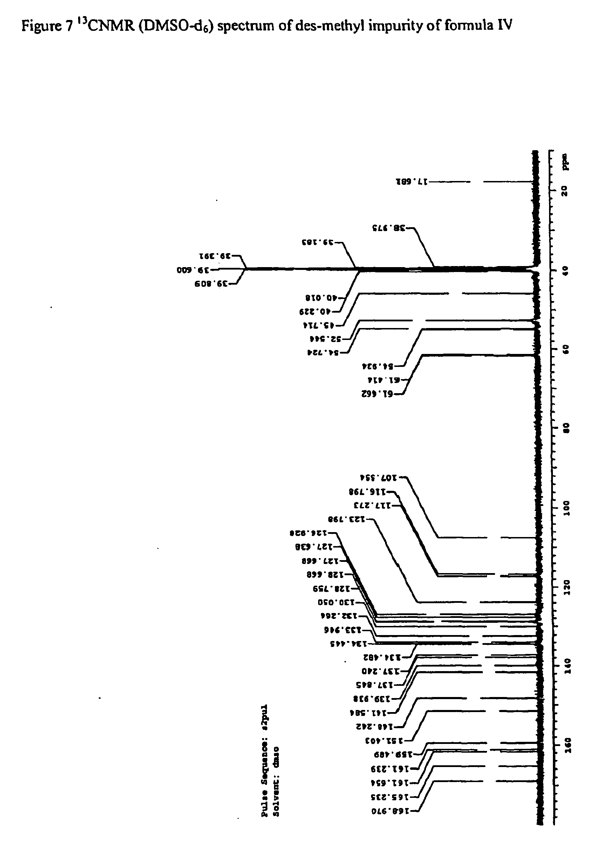

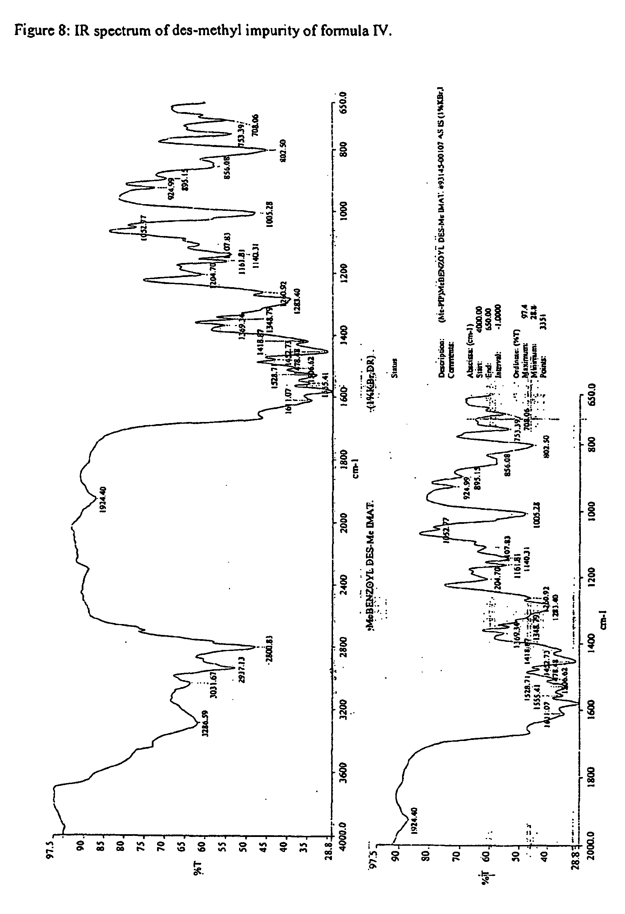

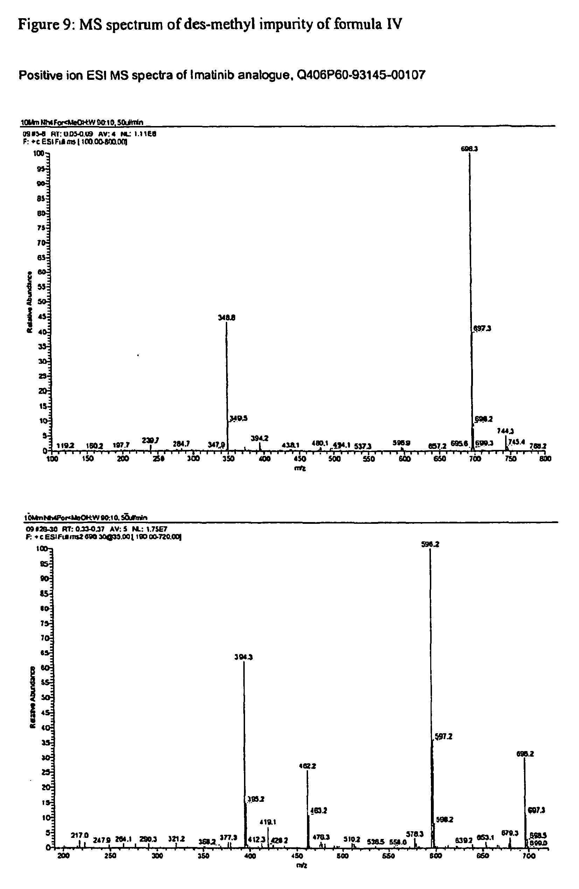

The des-methyl impurity of formula IV can be characterized by at least one of data selected from the group consisting of: ¹H NMR (DMSO-d₆) spectrum having peaks at about 2.141, 2.329, 2.305, 2.399, 3.463, 3.583, 7.322, 7.325, 7.923, and 10.159 ppm, ¹H NMR spectrum as depicted in figure 6, ¹³C NMR (DMSO-d₆) spectrum having peaks at about 45.71, 52.54, 54.73, 61.63, 61.42, 126.67, 126.69, 126.93, 128.76, 133.95, 134.48, 137.85, 139.94, 141.59, 165.24 and 168.97, ¹³C NMR spectrum as depicted in figure 7, IR spectrum having main peaks at about 1452, 1528, 1680 and 2937 cm⁻¹, IR spectrum as depicted in figure 8, MS spectrum having [MH]⁺ peak at about 696.g/mole, and MS spectrum as depicted in figure 9.

The above des-methyl compound of formula IV may be prepared by a process comprising reacting des-methyl imatinib of the following formula 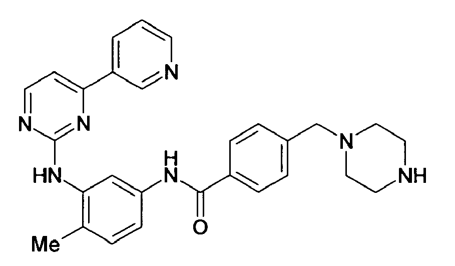 and the compound of formula V, 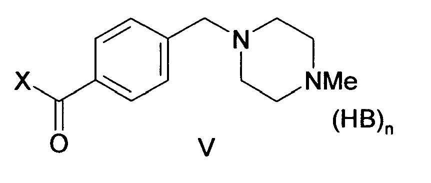 wherein X is a leaving group, HB is an acid, and n is 0, 1 or 2.

Preferably, X is Cl, Br or I, more preferably, X is Cl.

Preferably, HB is HCl, HI or HBr, more preferably, HB is HCl.

Preferably, n is either 0 or 2.

Typically, the reaction between des-methyl imatinib and the compound of formula V forms an acid ("HX") by-product, thus the reaction is done in the presence of a base that neutralizes this acid. The base can be an organic or inorganic base. Preferably, the organic base is an amine, more preferably, an aliphatic amine or aromatic amine. The aliphatic and the aromatic amines are preferably C₁-C₈ alkyl or aryl amines. Preferably, the aliphatic amine is selected from the group consisting of: triethylamine ("TEA"), di-isopropylamine ("DIPEA"), N-methylmorpholine, and mixtures thereof. Preferably, the aromatic amine is pyridine. Preferably, the inorganic base is an alkali metal base, more preferably, K₂CO₃, Na₂CO₃, NaHCO₃, KHCO₃ and mixtures thereof. Most preferably, the base is pyridine.

Preferably, the base is present in an amount of at least one mole equivalent per mole of the compound of formula V, depending on the nature of compound V. Preferably, when n is o, at least about 1 mole equivalent of base is sufficient. Preferably, if n is 1, at least about 2 mole equivalent of base should be used, and if n is 2, at least about 3 mole equivalent of base should be used.

Typically, the reaction is done in the presence of a solvent. Preferably, the solvent and des-methyl imatinib are combined to obtain a mixture. The solvent can be an organic solvent or the base itself (neat reagent). Preferably, the organic solvent is selected from the group consisitng of: tetrahydrofuran ("THF"), methyltetrahydrofuran ("MeTHF"), dioxolane, dichloromethane ("DCM"), dimethylformamide ("DMF"), dimethylacetamide ("DMA"), dimethylsulfoxide ("DMSO"), toluene , and mixtures thereof. When an organic solvent is used the solution also comprises an additional base.

Preferably, the solution is then combined with the compound of formula V, providing a reaction mixture. Preferably, the compound of formula V is added to the solution. Preferably, the addition is done at a temperature of about -10°C to about - 25°C, more preferably, at about 0°C to about 5°C, most preferably at about 3°C.

Preferably, the reaction mixture is allowed to warm to about 15°C to about 25°C followed by adding an additional amount of solvent, if required to make the less viscous.

The reaction mixture may be kept at such temperature for a sufficient time to allow the formation of the des-methyl compound of formula IV, preferably about 3 hours to about 10 hours, more preferably about 4 hours to about 6 hours. Preferably, the second mixture, wherein an additional amount of solvent has been added, is kept at this temperature for about 3 hours to about 10 hours preferably about 6 hours.

The obtained des-methyl compound of formula IV may then be recovered by any method known in the art, such as extractions and drying.

The des-methyl compound of formula IV can then be used to test the purity of imatinib. In one embodiment, the present invention encompasses a process of determining the presence of the des-methyl compound of formula IV in Imatinib by a process comprising carrying out HPLC or TLC with the des-methyl compound of formula IV as a reference marker.

Preferably, the method comprsises (a) measuring by HPLC or TLC the relative retention time (referred to as RRT, or RRF, respectively) corresponding to the desmethyl compound of formula IV in a reference marker sample; (b) determining by HPLC or TLC the relative retention time corresponding to the des-methyl compound of formula IV in a sample comprising the des-methyl compound of formula IV and imatinib; and (c) determining the relative retention time of the des-methyl compound of formula IV in the sample by comparing the relative retention time (RRT or RRF) of step (a) to the RRT or RRF of step (b).

In another embodiment, the present invention encompasses a process of determining the amount of the des-methyl compound of formula IV in a sample comprising the des-methyl compound of formula IV and imatinib by a process comprising carrying out HPLC with the des-methyl compound of formula IV as a reference standard.

Preferably, the above process comprises: (a) measuring by HPLC the area under a peak corresponding to the des-methyl compound of formula IV in a reference standard comprising a known amount of the des-methyl compound of formula IV; (b) measuring by HPLC the area under a peak corresponding to des-methyl compound of formula IV in a sample comprising des-methyl compound of formula IV and imatinib; and (c) determining the amount of the des-methyl compound of formula IV in the sample by comparing the area of step (a) to the area of step (b).

The HPLC method used to make the above analysis is, preferably, the same method used to measure the content of des-methyl imatinib and des-methyl imatinib mesylate.

Imatinib can be purifed from the des-methyl compound of formula IV when converted to imatinib mesylate. The purification can be done, for example according to the process disclosed in commonly assigned U.S. Application No. 11/796,573, incorporated herein by reference.

The process comprises providing a solution of imatinib mesylate and a mixture of water and ethanol; and precipitating by maintaining the solution at a temperature of about 0°C to about -30°C to obtain a suspension containing imatinib mesylate.

The obtained imatinib mesylate has a sufficient low amount of the des-methyl compound of formula IV, preferably, less than about 0.15% of the des-methyl impurity IV, more preferably less than about 0.10% of the des-methyl impurity IV. Typically, the measurement of the content of the des-methyl impurity of formula IV in imatinib mesylate is by area % units, preferably by HPLC.

The obtained Imatinib mesylate can be used for preparing pharmaceutical compositions.

In one embodiment, the present invention encompasses a pharmaceutical composition comprising imatinib mesylate having less than about 0.09% by HPLC area percent units of desmethyl-imatinib mesylate and at least one pharmaceutically acceptable excipient.

In another embodiment, the present invention encompasses a process for preparing the pharmaceutical composition, comprising combining imatinib mesylate having less than about 0.09% by HPLC area percent units of desmethyl-imatinib mesylate and the pharmaceutically acceptable excipient.

In yet another embodiment, the present invention encompasses the use of imatinib mesylate having less than about 0.09% by HPLC area percent units of desmethyl-imatinib mesylate in the manufacture of a pharmaceutical composition for the treatment of various types of cancer, atherosclerosis, thrombosis, restenosis, or fibrosis.

"Therapeutically effective amount" means the amount of the purified imatinib mesylate, when administered to a patient for treating a disease or other undesirable medical condition, is sufficient to have a beneficial effect with respect to that disease or condition. The "therapeutically effective amount" will vary depending on the purity, the disease or condition and its severity, and the age, weight, etc. of the patient to be treated. Determining the therapeutically effective amount of a given pure imatinib mesylate is within the ordinary skill of the art, and requires no more than routine experimentation.

Pharmaceutical formulations of the present invention contain the purified imatinib mesylate produced by the processes of the present invention. In addition to the active ingredient(s), the pharmaceutical formulations of the present invention may contain one or more excipients. Excipients are added to the formulation for a variety of purposes.

Diluents may be added to the formulations of a present invention. Diluents increase the bulk of a solid pharmaceutical composition, and may make a pharmaceutical dosage for containing the composition easier for the patient and caregiver to handle. Diluents for solid compositions include, for example, microcrystalline cellulose (e.g., AVICEL®, microfine cellulose, lactose, starch, pregelatinized starch, calcium carbonate, calcium sulfate, sugar, dextrates, dextrin, dextrose, dibasic calcium phosphate, dehydrate, tribasic calcium phosphate, kaolin, magnesium carbonate, magnesium oxide, maltodextrin, mannitol, polymethacrylates (e.g., EUDRAGIT®), potassium chloride, powdered cellulose, sodium chloride, sorbitol, and talc.

Solid pharmaceutical compositions that are compacted into dosage form, such as a tablet, may include excipients whose functions include helping to bind the active ingredient and other excipients together after compression. Binders for solid pharmaceutical compositions include acacia, alginic acid, carbomer (e.g., carbopol), carboxymethylcellulose sodium, dextrin, ethyl cellulose, gelatine, guar gum, hydrogenated vegetable oil, hydroxyethyl cellulose, hydroxypropyl cellulose (e.g., KLUCEL®), hydroxypropyl methyl cellulose (e.g., METHOCEL®), liquid glucose, magnesium aluminium silicate, maltodextrin, methylcellulose, polymethacrylates, povidone (e.g., KOLLIDON® PALSDONE®), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g., AC-DI-SOL®, PRIMELOSE®), colloidal silicon dioxide, croscarmellose sodium, crospovidone (e.g., KOLLIDON®. POLYPLASDONE®), guar gum, magnesium aluminium silicate, methyl cellulose, microcrystalline cellulose, polacrilin potassium, powdered cellulose, pregelatinized starch, sodium alginate, sodium starch glycolate (e.g., EXPLOTAB®), and starch.

Glidants can be added to improve the flowability of a non-compacted solid composition, and to improve the accuracy of dosing. Excipients that may function as glidants include colloidal silicon dioxide, magnesium trisilicate, powdered cellulose, starch, talc, and tribasic calcium phosphate.

When a dosage form such as tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion, and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavouring agents and flavour enhancers make the dosage form more palatable to the patient. Common flavouring agents and flavour enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance, and/or facilitate patient identification of the product and unit dosage level.

In liquid pharmaceutical compositions prepared using purified Imatinib mesylate produced by the processes of the present invention, Imatinib mesylate and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatine guar gum, hydroxyethyl cellulose, hydroxypropyl cellulose, hydroxpropyl methyl cellulose, maltodextrin, polyvinyl alcohol, povidone, propylene carbonate, propylene glycol alginate, sodium alginate, sodium starch glycolate, starch tragacanth, and xantham gum.

Sweetening agents such as sorbitol, saccharin, sodium saccharin, sucrose, aspartame, fructose, mannitol, and invert sugar may be added to improve the taste.

Preservatives and chelating agents such as alcohol, sodium benzoate, butylated hydroxyl toluene, butylated, hydroxyanisole, and ethylenediamine tetraacetic acid may be added at levels safe for ingestion to improve storage stability.

A liquid composition may also contain a buffer such as gluconic acid, lactic acid, citric acid or acetic acid, sodium gluconate, sodium lactate, sodium citrate, or sodium acetate. Selection of excipients and the amounts used may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant, and ophthalmic, administration. Although the most suitable administration in any given case will depend on the nature and severity of the condition being treated, the most preferred route of the present invention is oral.

The dosages may be conveniently presented in unit dosage form, and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The oral dosage form of the present invention is preferably in the form of an oral capsule having a dosage of about 10 mg to about 160 mg, more preferably from about 20 mg to about 80 mg, and most preferably capsules of 20, 40, 60, and 80 mg. Daily dosage may include 1, 2, or more capsules per day.

The dosage form of the present invention may be a capsule containing the composition, preferably a powdered or granulated solid composition of the invention, within either a hard or soft shell. The shell may be made from gelatin, and, optionally, contain a plasticizer such as glycerine and sorbitol, and an opacifying agent or colorant.

A composition for tableting or capsule filling may be prepared by wet granulation. In wet granulation, some or all of the active ingredients and excipients in powder form are blended, and then further mixed in the presence of a liquid, typically water, that causes the powders to clump into granules. The granulate is screened and/or milled, dried, and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet, and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules.

Excipients that are particularly well suited for direct compression tableting include microcrystalline cellulose, spray dried lactose, dicalcium phosphate dihydrate, and colloidal silica. The proper use of these and other excipients in direct compression tableting is know to those in the art with experience and skill in particular formulation challenges of direct compression tableting.

A capsule filling of the present invention may comprise any of the aforementioned blends and granulates that were described with reference to tableting, however, they are not subjected to a final tableting step.

The active ingredient and excipients may be formulated into compositions and dosage forms according to methods know in the art.

Having described the invention with reference to certain preferred embodiments, other embodiments will become apparent to one skilled in the art from consideration of the specification. The invention is further defined by reference to the following examples describing in detail the preparation of the composition and methods of use of the invention. It will be apparent to those skilled in the art that many modifications, both to materials and methods, may be practiced without departing from the scope of the invention.

### EXAMPLES

### HPLC METHODS

Analytical method for determination of the desmethyl impurity of formula II in the compound of formula I.

| | | | | |
|---|---|---|---|---|
| **Column & Packing** | **:** | YMC-Pack ODS-AQ; 5 µm, 250 x 4.6 mm (C.P.S. Analytica Part. No. AQ12S05-2546WT) or equivalent; | | |
| **Mobile Phase A** | **:** | 20mM 1-Butanesulfonic acid Sodium Salt + 10mM KH₂PO₄ to pH 2.5 with 85% H₃PO₄. | | |
| **Mobile Phase B** | **:** | Acetonitrile. | | |

| **Gradient** | **:** | Time (min) | Mobile Phase A (%) | Mobile Phase B (%) |
|---|---|---|---|---|
| | | 0 | 90 | 10 |
| | | 5 | 90 | 10 |
| | | 20 | 65 | 35 |
| | | 25 | 50 | 50 |
| | | 40 | 50 | 50 |
| **Run Time** | **:** | 40 minutes. | | |
| **Post Time** | **:** | 10 minutes. | | |
| **Flow Rate** | **:** | 1.0 mL/min. | | |
| **Detector** | **:** | λ = 230 nm. | | |
| **Column temperature :** | | 60 °C. | | |
| **Injection Volume** | **:** | 5 µL. | | |
| **Diluent** | **:** | Water. | | |

Typical retention times are:

| Compound | Retention Time (minutes) | Relative Retention Time |
|---|---|---|
| Compound (II) | 6.2 | 0.81 |
| Compound (I) | 7.6 | 1.00 |

| | | |
|---|---|---|
| The detection limit is 0.01 %. | | |

Analytical method for determination of the desmethyl imatinib impurity in imatinib

| | | | | |
|---|---|---|---|---|
| **Column & Packing** | **:** | YMC-Pack ODS-AQ; 5 µm, 250 x 4.6 mm (Part. No. AQ12S05-2546WT) or equivalent; | | |
| **Mobile Phase A** | **:** | 25mM 1-Butanesulfonic Acid Sodium Salt + 25mM KH₂PO₄ in Water to pH 2.3 with 85% H₃PO₄. | | |
| **Mobile Phase B** | **:** | Acetonitrile/Methanol/Tetrahydrofuran 70:10:20 (v/v/v). | | |

| **Gradient** | **:** | Time (min) | Mobile Phase A (%) | Mobile Phase B |
|---|---|---|---|---|
| | | | | (%) |
| | | 0 | 88 | 12 |
| | | 5 | 88 | 12 |
| | | 30 | 76 | 2 |
| | | 50 | 50 | 50 |
| | | 60 | 50 | 50 |
| **Run Time** | **:** | 60 minutes. | | |
| **Post Time** | **:** | 15 minutes. | | |
| **Integration Time** | **:** | 55 minutes. | | |
| **Flow Rate** | **:** | 0.9 mL/min. | | |
| **Detector** | **:** | λ = 235 nm. | | |
| **Column temperature :** | | 60 °c. | | |
| **Injection Volume** | **:** | 5 µL. | | |
| **Diluent** | **:** | Mobile Phase A/ Mobile Phase B 8:2 (v/v). | | |

Typical retention times are:

| Compound | Retention Time (minutes) | Relative Retention Time |
|---|---|---|
| Compound (III) | 23.8 | 0.95 |
| Compound (IV) | 24.1 | 0.96 |
| Imatinib | 25.1 | 1.00 |

| | | |
|---|---|---|
| The detection limit is 0.01%. | | |

### PXRD

PXRD diffraction was performed on X-Ray powder diffractometer: Philips X'pert Pro powder diffractometer, CuK_{α} radiation, λ = 1.5418 Å. Single-point detector; scan rate 3°/min. and multi-channel X'Celerator detector active length (2 theta) = 2.122°, ;scan rate 6°/min laboratory temperature 22-25°C.

### DSC ANALYSIS

DSC measurements were performed on Differential Scanning Calorimeter DSC823e (Mettler Toledo). Al crucibles 40 µl with PIN were used for sample preparation. Usual weight of sample was 1 -5 mg. Nitrogen as purging gas; 50ml/min.
Program 1: temperature range 50°C - 100 °C, 10°C/min than 100 °C - 250 °C, 40 °C/min.
Program 2: temperature range 50°C - 250 °C, 10°C/min

### Example 1: Preparation of crystalline Imatinib base of the present invention

To a solution of N-(5-amino-2-methylphenyl)-4-(3-pyridyl)-2-pyridineamine (80g) in pyridine (400g) was added 4-[(4-methyl-1-piperazinyl)methyl]benzoyl chloride dihydrochloride (1.1eq) at 0°C. The reaction was kept under stirring at 15-20°C for 1 h, and then water (400mL) was added. The mixture was heated up to 40°C, then 26% NH₄OH (200g) and water (900g) were added. The reaction mixture was kept under stirring at room temperature overnight. The solid was filtered off, washed with water and dried at 45°C under vacuum for 3-4 h. Imatinib was obtained as a yellowish powder (135g, 95% yield, >98% purity).

### Example 2: Preparation of crystalline Imatinib base of the present invention

To a suspension of 4-[(4-methyl-1-piperazinyl)methyl]benzoic acid (84g) in pyridine (400g)was added SOCl₂ (44.8g, 1.05eq) is added and the mixture is kept under stirring at 30-50°C for 1-2 h at 0°C. After cooling to 0°C, N-(5-amino-2-methylphenyl)-4-(3-pyridyl)-2-pyridineamine (80g) was added. The reaction was kept under stirring at 15-20 °C for 1 h, and then water (400mL) was added. The mixture
was heated up to 40°C, then 26% NH₄OH (200g) and water (900mL) were added. The reaction mixture was kept under stirring at room temperature overnight. The solid was filtered off, washed with water and dried at 45°C under vacuum overnight. Crystalline Imatinib base of the present invention was obtained as a yellowish powder (125g, 88% yield, >98% purity).

### Example 3: Preparation of crystalline Imatinib base of the present invention

To a suspension of 4-[(4-methyl-1-piperazinyl)methyl]benzoic acid dihydrochloride (30g) in pyridine (100g) was added SOCl₂ (11.5g, 1.05eq) at 20°C, and the mixture was kept under stirring at 45-50°C for 1-2 h. After cooling to 0°C, N-(5-amino-2-methylphenyl)-4-(3-pyridyl)-2-pyridineamine (20g) were added. The reaction was kept under stirring at 15-25°C for 1 h, and then water (100mL) was added. The mixture was heated up to 40°C, then 26% NH₄OH (50g) and water (225mL) were added. The reaction mixture was kept under stirring at room temperature for overnight. The solid was filtered off, washed with water and dried at 45°C under vacuum for overnight. Crystalline Imatinib base of the present invention was obtained as a yellowish powder (32g, 90% yield, >98% purity).

### Example 4: Preparation of crystalline Imatinib base of the present invention

28% NH₃ (30 mL) was added at 40°C to a solution of imatinib mesylate (60g) in a mixture of pyridine (140mL) and water (70mL). The solution was kept under stirring at 40°C until precipitation of imatinib base occurred. Additional amount of water (490mL) was added at 40°C, then the mixture was allowed to spontaneously reach room temperature and it was kept under stirring for 15h. The solid was filtered off, washed with water and dried under vacuum at 40°C overnight. Crystalline Imatinib base of the present invention was obtained as a yellowish solid (50g, 90% yield).

### Example 5: Preparation of crystalline Imatinib base of the present invention

To a solution of Imatinib base (94g) in pyridine (376g) and water (188g) at 40-50°C, water (1300g) was added. The mixture was kept under stirring at 15-20°C overnight, then the solid was filtered off, washed with water and dried at 40°C under vacuum for 16 h. Imatinib base was obtained as a yellowish solid (99.6g, >99% purity).

### Example 6: Preparation of crystalline Imatinib base of the present invention

37% HCl (9mL) was added to a suspension of imatinib base (50g) in a mixture of pyridine (140mL) and water (70mL). The solution was heated up to 40°C, treated with charcoal and filtered. 28% NH₃ (30 mL) was added to the filtrate (pH = 9.3) at 40°C and the solution was kept under stirring at 40°C until precipitation of imatinib base occurred. Additional of water (490mL) was added at 40°C, then the mixture was allowed to spontaneously reach room temperature and it was kept under stirring for 15h. The solid was filtered off, washed with water and dried under vacuum at 40°C overnight. Crystalline Imatinib base of the present invention was obtained as a yellowish solid (50g, 90% yield).

### Example 7: conversion of Imatinib base to imatinib mesylate according to WO No 1999/03854

4-[(4-Methyl-1-piperazinyl)methyl]-N-[4-methyl-3-[[4-(3-pyridinyl)-2-pyrimidinyl]aminophenyl] benzamide (98.6g) was added to EtOH (1.4L). To the suspension methanesulfonic acid (19.2g) was added dropwise. The solution was heated under reflux for 20 min and then filtered clear at 65°C. The filtrate was evaporated down to 50% and the residue filtered off at 25°C (filtered material A). The mother liquor were evaporated to dryness. This residue and filtered material A were suspended in EtOH (2.2L) and dissolved under reflux with addition of water (30mL). The solution was cooled down and kept overnight at 25°C. The solid is filtered off and dried at 65°C.

### Example 8: Preparation of amorphous imatinib base

Imatinib base (500 mg) was dissolved in 1,4-dioxane (10 ml) at 90°C. The solution was allowed to cool to 25°C and put to the freezer at -30°C where the solution was frozen. The frozen solution was transferred to the lyophylisator and vacuum 1 mBar was applied, which provided the freeze drying of 1,4-dioxane affording amorphous imatinib base.

### Example 9: Crystallizations of the compound of formula I (n=2)

A suspension of compound I in a mixture of water (320mL) and IPA (420mL) was heated at 75°C in order to obtain a clear solution. Under vigorous stirring, the reaction mixture was cooled down to 20-25°C in 1 hours, then to 0-3°C in 1-1.5 h and kept under stirring at this temperature for 1-2 h. The solid was filtered off and the solid cake was washed with IPA (180mL). The product was dried at 60-65°C under vacuum for 15 h. Pure compound (I) was obtained as a white solid (131g).

| **Sample of compound of formula I** | **Level of desmethyl impurity of formula II (area percent)** |
|---|---|
| Starting material (before crystallization) | 0.42% |
| Product obtained after first crystallization | 0.13% |
| Product obtained after second crystallization | Less than 0.04% |

### Example 10: Synthesis of desmethyl impurity (II)

A mixture of 4-chloromethylbenzoic acid (10g, 58.6 mmol) and piperazine (20g, 232mmol) in *n*-BuOH (100g) was heated at 50°C for 3h, then kept at room temperature overnight. The solid was filtered off, washed with n-BuOH and dried at 70°C overnight. Desmethyl impurity (II) was obtained as a white solid (17.5g, 86.6% purity).

### Example 11: Synthesis of desmethyl imatinib Synthesis of 4-chloromethyl-N-[methyl-3-(4-pyridin-3-yl-pyrimidin-2ylamino)-phenyl]-benzamide

4-Chloromethylbenzoyl chloride (15g) was added to a mixture of N-(5-amino-2-methylphenyl)-4-(3-pyridyl)-2-pyridineamine (18.5g), K₂CO₃ (20g) in THF (370g) at 2-3°C. The mixture was kept under stirring at 2-7°C for 1 h, then at 15-20°C for additional 3 h. Water (700g) was added and the suspension was kept under stirring at 15-20°C for 1h. The solid was filtered off, washed with water and dried at 65°C under vacuum for 15 h to furnish the title product (27g, 94% yield).

Cp9665 (14g) was added portion wise in 15 min to a solution of piperazine (28.4g) in EtOH (22g) and water (27.5g) at 50°C. The reaction mixture was refluxed for 2h, then cooled down to room temperature and kept under stirring overnight. The solid was filtered off, washed with a mixture of water (13.75g) and EtOH (11g) and taken up with water (400g) and AcOEt (100g). After addition of 28% NH3 (25g), the mixture was kept under stirring overnight. The solid was filtered off, washed with water and dried at 70°C under vacuum for 15 h to furnish the desired product (12g).

### Example 12: Preparation of crystalline Imatinib base having less than 0.09% of des-methyl imatinib

37% HCl (9mL) was added to a suspension of imatinib base (50g, desmethyl 0.12%) in a mixture of pyridine (140mL) and water (70mL). The solution was heated up to 40°C, treated with charcoal and filtered. 28% NH3 (30 mL) was added to the filtrate (pH = 9.3) at 40°C and the solution was kept under stirring at 40°C until precipitation of imatinib base occurred. Additional of water (490mL) was added at 40°C, then the mixture was allowed to spontaneously reach room temperature and it was kept under stirring for 15h. The solid was filtered off, washed with water and dried under vacuum at 40°C overnight. Imatinib base was obtained as a yellowish solid (50g, 90% yield, 0.08% desmethyl content).

This crystallization, however, didn't succeed in decreasing the level of the desmethyl impurity of formula IV. The starting imatinib base had 0.55% of the desmethyl impurity of formula IV, and so did the crystallized imatinib base.

### Example 13: Preparation of crystalline Imatinib base having less than 0.09% of des-methyl imatinib

To a suspension of compound I (n=2, X-=Cl) (20g, containing 0.13% of the des-methyl impurity II) in toluene (35mL) and DMF (1mL) under N2 at 60°C, (20g) was added over a period of 1 h SOCl₂. The mixture was kept under stirring at 62°C for 20 h. After cooling at 20°C, toluene (20mL) was added and the mixture was stirred for 0.5 h. The solid was filtered off, washed with toluene (50 mL) and dried at 65°C under vacuum for 15h. The product was obtained as a white powder (21g). To a solution of the amine of formula III (R=H) (1.5kg,) in pyridine (8.25L) at 0°C, was added as solid in one portion under N2 compound I (n=2; X=Cl) (2.69kg). The temperature increased spontaneously to 5-10°C. The reaction was kept under stirring at 15-20°C for 2 h, then water (8.25L) was added allowing the temperature to increase, then the mixture was heated up to 35-40°C. Charcoal Norit S2 (75g) was added and the mixture was stirred at 40°C for 30min, then filtered over dicalite bed. The panel was washed with water (6L). 28% NH4OH (4.05L) was added to the filtrate and the mixture was kept under stirring for 15-30 min, in order to obtain the product precipitation (mixture pH = 9.4). Water (15.2L) was added and the reaction mixture was kept under stirring at 20°C for 15 h. The solid was filtered off, washed with water and dried at 40°C under vacuum for 8 h. Imatinib was obtained as a yellowish powder (2.98kg, 93% yield, desmethyl imatinib 0.08%).

### Example 14 : Synthesis of desmethyl imatinib mesylate

MeSO₃H (2.4g) was added to a solution of desmethyl imatinib (12g) in MeOH (240g) at 60°C. The solvent was evaporated under vacuum and the residue was taken up with EtOH (72g) and AcOEt (360g). The mixture was stirred at room temperature overnight, then the solid was filtered off, washed with AcOEt and dried under vacuum at 75°C to furnish the title product (13.9g).

### Example 15: Synthesis of desmethyl imatinib derivative (IV)

4-[(4-methyl-1-piperazinyl)methyl]benzoyl chloride dihydrochloride (4g) was added to a solution of desmethyl imatinib (III) (5g) in pyridine (25mL) at 3°C. The mixture was allowed to reach room temperature, then additional pyridine (25mL) was added and the mixture was stirred at room temperature for 6h. Water (50mL) and 28% NH3 were added and the mixture was evaporated to dryness under vacuum. The residue was taken up with DCM and water. The organic phase was separated and evaporated to dryness to furnish the title compound as a yellowish powder (4g).

### Example 16: The correlation between the levels of the desmethyl impurities in the different products

| **Level of the desmethyl impurity of formula II in the compound of formula I (n=2) (area percent)** | **Level of the desmethyl imatinib in imatinib (area percent)** | **Level of the desmethyl imurity of formula IV in imatinib (area percent)** |
|---|---|---|
| | | |
| 0.42% | 0.13% | 0.33%* |
| 0.22% | 0.04% | 0.06%* |
| 0.10% | Less than 0.04% | Not detected |
| 0.13% | 0.08% | Not detected |

| | | |
|---|---|---|
| * some of the des-methyl imatinib impurity converted to impurity IV, thus their total amount is more than 0.09%. | | |

### Example 17: Analysis of Gleevec^{®} tablet

The coating was peeled off from 5 tablets and the remainder was milled in a mortar. 20 mg of the powder were taken up with 4 mL of mobile phase B and 16 mL of mobile phase A. The mixture was sonicated for 5 min and then filtered. The filtrate was injected in HPLC.

| **GLEEVEC^{®}** | **Levels of N-Des- Methyl Imatinib Mesylate (area percent)** | **Levels of Imatinib Mesylate (area percent)** |
|---|---|---|
| **GLEEVEC (EU) Tablets 400 mg Lot: F0004 Exp. Date: Feb. 2007** | 0.09% | 99.91% |
| **GLIVEC (JP) Tablets 100 mg Lot: S0016 Exp.date: July 2009** | 0.10% | 99.90% |

### Example 18: Crystallization of Imatinib mesylate-Impurity Level is Maintained

Imatinib mesylate (4.2 g, containing 0.08% of desmethyl imatinib) was added to MeOH (10.5 mL) and the mixture was heated at 60°C. The solution was allowed to cool to 20°C under stirring. After 30 min stirring at 20°C, the solid was filtered off and dried under vacuum at 100°C to furnish imatinib mesylate (3.8g, containing 0.08% of desmethyl imatinib).

### Example 19: Crystallization of Imatinib mesylate-Impurity Level is Maintained

Imatinib mesylate (4.2 g, containing 0.39% of desmethyl imatinib) was added to MeOH (10.5 mL) and the mixture was heated at 60°C. The solution was allowed to cool to 20°C under stirring. After 30 min stirring at 20°C, the solid was filtered off and dried under vacuum at 100°C to furnish imatinib mesylate (3.8g, containing 0.38% of desmethyl imatinib).

### Example 20: Purification of imatinib from des-methyl impurity of formula IV

Imatinib base (60 g; 0.1216 mole) was suspended in 1200 ml of Ethanol and stirred. Reactor was kept under flow of nitrogen during all of the experiment (6 litres per hour). Then, 24 ml of water was added to the suspension and the temperature was adjusted at -15°C. An ethanolic solution of methanesulfonic acid (79.8 ml 10% V/V; 0.1213 mole) was added during 2 minutes to the reaction mixture. Temperature of the solution was set at -10°C during 10 minutes, imatinib base was dissolved and seeding crystalline material (2 g) was added. The crystallization process was continued under stirring for 190 minutes and temperature was continuously increased to -5°C. The suspension was stored overnight in a freezer at approx. -27°C. Than, suspension was diluted by 1000 ml TBME, filtered by nitrogen pressure and obtained crystalline portion was washed with 400 ml TBME. The resulted crystalline form was dried by flow of nitrogen through the filter to remove free ethanol. Ethanol content was about 7.5 %. (Yield was 67.95 g; 85%)

| | Des-methyl impurity IV |
|---|---|
| Imatinib base | 0.51 |
| Imatinib mesylate | 0.36 |

Further aspects and features of the present invention are set out in the following numbered clauses:
1. A crystalline Imatinib base characterized by data selected from the group consisting of: a powder XRD pattern having any five peaks selected from the list consisting of peaks at about: 6.4, 8.1, 10.2, 12.8, 16.1, 19.4, 20.4, 21.7, 22.1, 25.8 and 26.7 ± 0.2 degrees two-theta; a solid-state ¹³C NMR spectrum with signals at about 159.6, 146.7, 136.8 and 132.4 ± 0.2 ppm; a solid-state ¹³C NMR spectrum having chemical shift differences between the signal exhibiting the lowest chemical shift and another in the chemical shift range of 100 to 180ppm of about 51.2, 38.3, 28.4 and 24.0 ± 0.1 ppm.
2. The crystalline imatinib base of clause 1, wherein the crystalline imatinib base is characterized by data selected from the group consisting of: a powder XRD depicted in figure 3, a solid state ¹³C NMR spectrum depicted in figure 1, and a solid state ¹³C NMR spectrum depicted in figure 2.
3. The crystalline imatinib base of clause 1 or 2, further characterized by data selected from the group consisting of: a powder XRD pattern having peaks at about 8.1, 10.2, 12.8, 16.1, and 19.4 ± 0.2 degrees two-theta; a powder XRD pattern having peaks at about 6.4, 8.1, 10.2, 19.4, 20.4 and 25.8 ± 0.2 degrees two-theta; a powder XRD pattern with peaks at about 17.3, 20.4, 21.1, and 25.8 ± 0.2 degrees two-theta; a powder XRD pattern with peaks at about 6.4, 21.7, 22.1 and 26.7± 0.2 degrees two-theta; a solid-state ¹³C NMR spectrum with signals at about 125.8 and 108.4 ± 0.2 ppm; a DSC curve having 2 peaks, the first peak is an endothermic peak at 97.6°C and a second peak is an endothermic peak at 210.5 °C; and a DSC curve as depicted in figure 4.
4. The crystalline imatinib base of any one of clauses 1-3, wherein the crystalline imatinib base is a pyridine solvate of imatinib base.
5. The crystalline imatinib base of clause 4, wherein the pyridine solvate is a hemi-pyridine solvate.
6. The crystalline imatinib base of any one of clauses 4-5, wherein the pyridine content is of about 7% (w/w) as measured by GC.
7. The crystalline imatinib base of any one of clauses 1-6, wherein the imatinib base has less than about 20% of crystalline form I of Imatinib base as measured by PXRD or solid state C¹³ NMR.
8. A process for preparing crystalline imatinib base of any one of clauses 1-8 comprising reacting the amine of formula III, 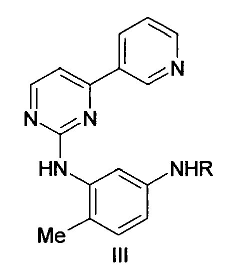 with a 4-[(4-methyl-1-piperazinyl)methyl]benzoyl derivative of formula V 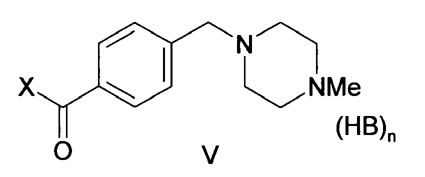 and an amount of about 2 to about 10 volumes of pyridine per gram of the compound of formula III, and recovering Imatinib base; wherein X is a leaving group selected from the group consisting of: Cl, Br; R is either H or a C₁₋₆ alkyl group, HB is an acid, and n= 0, 1, or 2.
9. The process of clause 8, wherein X is Cl and R is H.
10. A process for preparing the crystalline imatinib base of any one of clauses 1-8 comprising combining imatinib base or a salt thereof with pyridine to obtain a mixture, and crystallizing imatinib base from the mixture.
11. The process of clause 10, wherein crystallizing comprises preparing a solution containing imatinib base, pyridine and a solvent, and adding an anti-solvent to obtain a precipitate of said crystalline imatinib base.
12. The process of any one of clauses 10-11, wherein the solvent is water, a water-miscible organic solvent or mixtures thereof.
13. The process of clause 12, wherein the water-miscible organic solvent is selected from the group consisting of: dimethylformamide, dimethylacetamide, tetrahydrofuran, alcohol, acetone, acetonitrile, dioxane, dimethylsulfoxide, and mixtures thereof.
14. The process of clause 12, wherein the solvent is dimethylformamide, dimethylacetamide, tetrahydrofuran, or water.
15. The process of any one of clauses 10-14, wherein the solution is prepared by combining imatinib salt and an additional base to obtain imatinib base
16. The process of clause 15, wherein the additional base is an organic base, selected from the group consisting of a tertiary amine and an inorganic base is an alkali salt selected from the group consisting of potassium hydroxide, sodium or potassium carbonate, sodium or potassium bicarbonate or ammonia.
17. The process of any one of clauses 10-16, wherein the solution is provided by heating the combination of imatinib base or salt, the solvent, pyridine and optionally the additional base to a temperature of about 5°C to about 70°C.
18. The process of clause 17, wherein heating is to a temperature of about 40°C to about 50°C.
19. The process of any one of clauses 10-18, wherein the pyridine is present in the solution in an amount of about 2 to about 10 volumes per gram of Imatinib base or salt.
20. The process of any one of any one of clauses 10-19, further comprising cooling the slurry to a temperature of about 30°C to about 0°C for period of about 1 hour to about 24 hours.
21. A process for preparing Imatinib salt comprising converting the crystalline imatinib base of any one of clauses 8-21 to an imatinib salt.
22. Amorphous imatinib base.
23. The amorphous imatinib base of clause 22, wherein the amorphous Imatinib base can be characterized by the X-ray powder diffraction pattern depicted in Figure 5.
24. A process preparing an amorphous Imatinib base of clause 22 or 23 comprising lyophilizing a solution of imatinib base in 1,4-dioxane.
25. The process of clause 24, wherein the solution is provided by combining imatinib base and 1,4-dioxane, and heating the combination to a temperature of about 50°C to about 110°C.
26. The process of any one of any one of clauses 24-25, wherein the lyophilization process is carried out at a temperature of about 12°C to about 0°C.
27. The process of clause 26, wherein lyophilization is carried out at about 0.01 to about 100 mBar.
28. A process for preparing Imatinib salt comprising converting amorphous imatinib base to an imatinib salt.
29. An Imatinib mesylate of the following formula 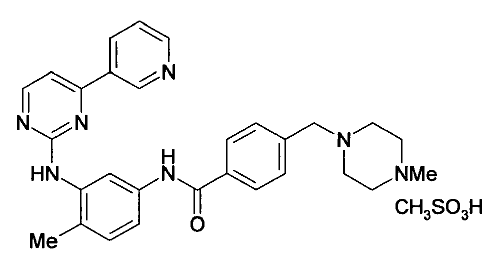 having less than about 0.09% by HPLC area percent units of desmethyl-imatinib mesylate of the following formula. 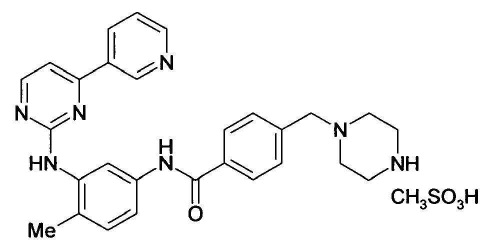
30. The Imatinib mesylate of clause 29, having less than 0.07% by area percent units of desmethyl-imatinib mesylate.
31. An Imatinib base of the following formula 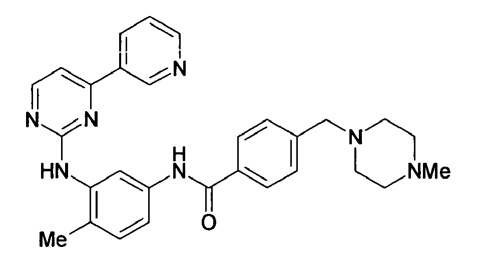 having less than about 0.09% by area HPLC percent units of desmethyl-imatinib of the following formula. 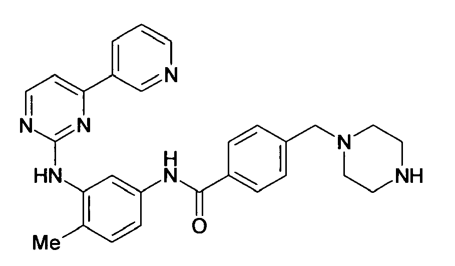
32. The Imatinib base of clause 31, having less than 0.07% by area HPLC percent units of desmethyl-imatinib base.
33. A process of preparing Imatinib having less than about 0.09% by area HPLC percent units of desmethyl-imatinib comprising:
   a) measuring the level of the desmethyl impurity of formula II 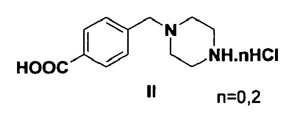 in at least one batch of the compound of formula I, 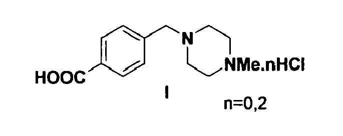
   b) selecting a batch of the compound of formula I having less than about 0.15% of the desmethyl impurity of formula II; and
   c) preparing imatinib with the selected batch of the compound of formula I, wherein n is either 0, 1 or 2.
34. The process of clause 33, wherein the measurement of the content of the desmethyl impurity of formula II by area HPLC percent units comprises
   a) combining a sample comprising of the compound of formula I and desmethyl impurity of formula II with water to obtain a solution;
   b) injecting the solution to a C18 reversed phase silica based HPLC column;
   c) eluting the sample from the column using a gradient eluent of a mixture of 1-butanesulfonic acid sodium salt, KH₂PO₄and H₃PO₄, (referred to as mobile phase A), and a mixture of acetonitrile, (referred to as mobile phase B), and
   d) measuring the content of the desmethyl impurity of formula II using a UV detector.
35. The process of clause 33 or 34, wherein the compound of formula I having less than about 0.15% area HPLC percent units of the desmethyl impurity of formula II is provided by a process comprising crystallizing the compound of formula I from a mixture of water and a C₁₋₃ alcohol.
36. The process of clause 35, wherein crystallization comprises providing a solution of the compound of formula I in a mixture of water and C₁₋₃ alcohol, and precipitating the compound of formula I.
37. The process of clause 35 or 36, wherein the solution is provided by combining the compound of formula I with a mixture of water and C₁₋₃ alcohol and heating the combination to a temperature of about 55°C to about 80°C.
38. The process of clause 37, wherein the temperature is of about 65 to about 75°C.
39. The process of any one of clauses 36- 38, wherein the C₁₋₃ alcohol is isopropanol ("IPA").
40. The process of any one of clauses 36-39, wherein the ratio of the C₁₋₃ alcohol and water in the mixture is of about 80: 20.
41. The process of any one of clauses 36-40, wherein the solution is cooled to a temperature of about 50°C to about -5°C.
42. The process of clause 41, wherein cooling is carried out step-wise comprising first cooling the solution to a temperature of about 35°C to about 15°C, and the second cooling step is to a temperature of about 5°C to about -5°C.
43. The process of clause 42, wherein the first cooling stage is done over a period of about 0.5 an hour to about 3 hours and the second cooling is done over a period of about 0.5 an hour to about 5 hours.
44. The process of any one of clauses 41-43, wherein the cooled solution is kept at such temperature for about 1 hour to about 5 hours.
45. The process of any one of clauses 33-44, wherein preparing imatinib as in step (c) comprises reacting the compound of formula I having less than about 0.15% by area HPLC percent units of the desmethyl impurity of formula II with the amine of formula III, 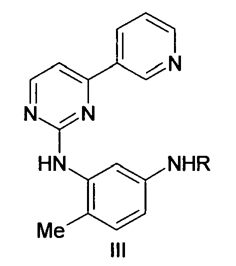 to obtain Imatinib having less than about 0.09% by area HPLC percent units of desmethyl imatinib.
46. The process of clause 45, further comprising crystallizing the obtained Imatinib.
47. The process of clause 45, further comprising preparing imatinib mesylate having less than about 0.09% area HPLC percent units of des-methyl imatinib by a process comprising measuring the level of desmethyl imatinib in at least one batch of imatinib, selecting a batch of imatinib having less than about 0.09% area HPLC percent units, and preparing imatinib mesylate with imatinib having less than about 0.09% area HPLC percent units.
48. A des-methyl compound of formula IV 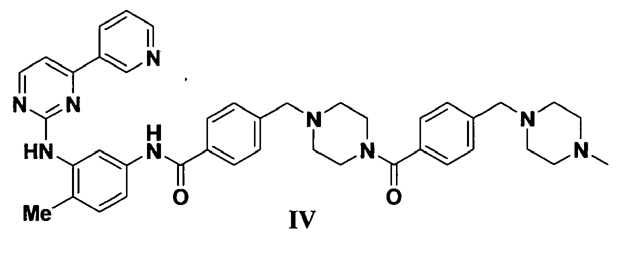
49. The des-methyl compound of formula IV having less about 0.15% area HPLC percent units of desmethyl imatinib.
50. The des-methyl compound of formula IV, characterized by at least one of data selected from the group consisting of: ¹H NMR (DMSO-d₆) spectrum having peaks at about 2.141, 2.329, 2.305, 2.399, 3.463, 3.583, 7.322, 7.325, 7.923, and 10.159 ppm, ¹H NMR spectrum as depicted in figure 6, ¹³C NMR (DMSO-d₆) spectrum having peaks at about 45.71, 52.54, 54.73, 61.63, 61.42, 126.67, 126.69, 126.93, 128.76, 133.95, 134.48, 137.85, 139.94, 141.59, 165.24 and 168.97, ¹³C NMR spectrum as depicted in figure 7, IR spectrum having main peaks at about 1452, 1528, 1680 and 2937 cm⁻¹, IR spectrum as depicted in figure 8, MS spctrun having [MH]⁺ peak at about 696.g/mole, and MS spectrum as depicted in figure 9.
51. A process of preparing the desmethyl compound of formula IV of clauses 48-50 comprising reacting des-methyl imatinib of the following formula 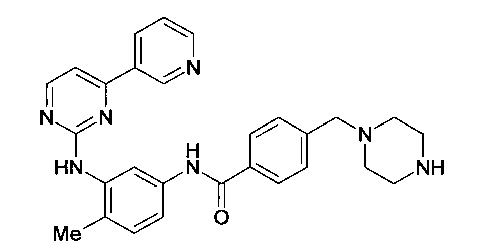 with the compound of formula V, 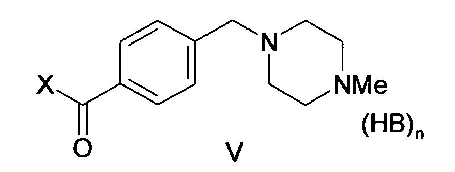 wherein X is a leaving group, HA is an acid, and n is 0, 1 or 2.
52. The process of clause 51, wherein X is Cl, HB is HCl, and n is either 0 or 2.
53. The process of any one of clauses 51-52, wherein a base is added to the reaction.
54. The process of clause 53, wherein the base is selected from the group consisting of an amine and an alkali metal base.
55. The process of any one of clauses 53-54, wherein the base is selected from the group consisting of: triethylamine ("TEA"), di-isopropylamine ("DIPEA"), N-methylmorpholine, mixtures thereof, K₂CO₃, Na₂CO₃, NaHCO₃, KHCO₃ and mixtures thereof.
56. The process of any one of clauses 53-55, wherein the amount of the base is at least one molar equivalent per mole of compound V.
57. The process of clause 51-56, wherein the reaction is done is a solvent selected from the group consisting of: tetrahydrofuran ("THF"), methyltetrahydrofuran ("MeTHF"), dioxolane, dichloromethane ("DCM"), dimethylformamide ("DMF"), dimethylacetamide ("DMA"), dimethylsulfoxide ("DMSO"), toluene, and mixtures thereof.
58. The process of any one of clauses 51-57, wherein the reaction mixture is allowed to warm to a temperature of about 15°C to about 25°C followed by adding an additional amount of solvent.
59. The imtanib compound of formula IV of clause 48-50, wherein des methyl compound of formula IV is a reference marker.
60. A process of determining the amount of the des methyl compound of formula IV is present in a sample comprising (a) measuring by HPLC the area under a peak corresponding to the des-methyl compound of formula IV in a reference standard comprising a known amount of the des-methyl compound of formula IV; (b) measuring by HPLC the area under a peak corresponding to des-methyl compound of formula IV in a sample comprising des-methyl compound of formula IV and imatinib; and (c) determining the amount of the des-methyl compound of formula IV in the sample by comparing the area of step (a) to the area of step (b).
61. A pharmaceutical composition comprising imatinib mesylate having less than about 0.09% area HPLC percent units of desmethyl-imatinib mesylate and at least one pharmaceutically acceptable excipient.
62. A process for preparing the pharmaceutical composition, comprising combining imatinib mesylate having less than about 0.09% area HPLC percent units of desmethyl-imatinib mesylate and the pharmaceutically acceptable excipient.

## Claims

1. Amorphous imatinib base.

2. Amorphous imatinib base according to claim 1, wherein the amorphous Imatinib base can be **characterized by** the X-ray powder diffraction pattern depicted in Figure 5.

3. Amorphous imatinib base according to claim 1 or claim 2 containing less than about 20%, preferably less than about 10%, and more preferably less than about 5% crystalline form as measured by area percentage XRD.

4. A process preparing an amorphous Imatinib base of any preceding claim comprising lyophilizing a solution of imatinib base in 1,4-dioxane.

5. A process according to claim 4, wherein the solution is provided by combining imatinib base and 1,4-dioxane, and heating the combination.

6. A process according to claim 5 wherein the heating is conducted to a temperature of about 50°C to about 110°C, and preferably about 90°C to about 101 °C.

7. A process according to claim 6 wherein the solution is cooled prior to the lyophilization process, preferably wherein the cooling is to a temperature of about 25°C to about 12°C.

8. A process according to any one of claims 4-7 wherein the lyophilization process is carried out at a temperature of about 12°C to about 0°C.

9. A process according to any of claims 4-8 wherein the lyophilization is carried out at about 0.01 to about 100 mBar, preferably about 0.1 to about 15 mBar, and more preferably at about 1 mBar.

10. A process for preparing Imatinib salt comprising converting amorphous imatinib base to an imatinib salt, preferably imatinib mesylate.

11. A process according to claim 10 wherein the amorphous imatinib base is prepared by a process according to any of claims 4-10.

12. Use of imatinib base according to any of claims 1-3 for the preparation of imatinib salt.
